# EUROPEAN PATENT SPECIFICATION

(11) **EP 3 493 854 B1**
(45) Date of publication and mention of the grant of the patent: **04.10.2023**
(21) Application number: 17754896.3
(22) Date of filing: 04.08.2017
(51) Int. Cl.: A61K 38/38, A61K 47/69, A61K 39/395, C07K 16/28, A61K 39/00, A61K 31/337, A61K 45/06, A61K 47/64, A61K 9/19, A61P 35/00, C07K 16/22, C07K 16/32

(54) **MODIFIED ANTIBODY-ALBUMIN NANOPARTICLE COMPLEXES FOR CANCER TREATMENT**
MODIFIZIERTE ANTIKÖRPER-ALBUMIN-NANOPARTIKELKOMPLEXE ZUR KREBSBEHANDLUNG
COMPLEXES DE NANOPARTICULES D'ANTICORPS-ALBUMINE MODIFIÉES POUR LE TRAITEMENT DU CANCER.

(30) Priority: 05.08.2016 US 201662371668 P; 18.10.2016 US 201662409830 P
(43) Date of publication of application: 12.06.2019
(73) Proprietor: Mayo Foundation for Medical Education and Research, Rochester, MN 55905 (US)
(72) Inventor: MARKOVIC, Svetomir N., Rochester, Minnesota 55905 (US); NEVALA, Wendy K., Rochester, Minnesota 55905 (US); BUTTERFIELD, John Thomas, Rochester, Minnesota 55905 (US); KNAUER, Daniel Joseph, Rochester, Minnesota 55905 (US)
(74) Representative: Fish & Richardson P.C.
(86) International application number: PCT/US2017/045643
(87) International publication number: WO 2018/027205

(56) References cited:
- EP-A1- 3 533 870
- WO-A1-2014/055415
- WO-A1-2015/191969
- WO-A1-2016/057554
- WO-A2-2006/034455
- WO-A2-2010/136492
- W. K. NEVALA ET AL: "Antibody-Targeted Chemotherapy for the Treatment of Melanoma", CANCER RESEARCH, vol. 76, no. 13, 1 July 2016 (2016-07-01), pages 3954-3964, XP55415276, US ISSN: 0008-5472, DOI: 10.1158/0008-5472.CAN-15-3131
- WENDY K ANNEVALA ET AL: "Abstract B77: Targeted nano-immune conjugates to melanoma: Preclinical testing of bevacizumab targeted nab-paclitaxel", CANCER IMMUNOL RES, vol. 3, 1 October 2015 (2015-10-01), pages B77-B77, XP55416235, DOI: 10.1158/2326-6074.TUMIMM14-B77 -& WENDY K NEVALA ET AL: "Targeted nano-immune conjugates to melanoma: Preclinical testing of bevacizumab targeted nab-paclitaxel", PROCEEDINGS OF THE AACR SPECIAL CONFERENCE: TUMOR IMMUNOLOGY AND IMMUNOTHERAPY: A NEW CHAPTER, 1 December 2014 (2014-12-01), XP55416238, Orlando, FL
- WENDY K. NEVALA ET AL: "Antibody-targeted paclitaxel loaded nanoparticles for the treatment of CD20+ B-cell lymphoma", SCIENTIFIC REPORTS, vol. 7, 5 April 2017 (2017-04-05), page 45682, XP55415269, DOI: 10.1038/srep45682

## Description

### CROSS-REFERENCE TO RELATED APPLICATIONS

This application claims the benefit of the priority date of U.S. Provisional Application Nos. 62/371,668, filed on August 5, 2016; and 62/409,830, filed on October 18, 2016.

### BACKGROUND

Chemotherapy remains a mainstay for systemic therapy for many types of cancer, including melanoma. Most chemotherapeutics are only slightly selective to tumor cells, and toxicity to healthy proliferating cells can be high (Allen TM. (2002) Cancer 2:750-763), often requiring dose reduction and even discontinuation of treatment. In theory, one way to overcome chemotherapy toxicity issues as well as improve drug efficacy is to target the chemotherapy drug to the tumor using antibodies that are specific for proteins selectively expressed (or overexpressed) by tumor cells, thereby altering the biodistribution of the chemotherapy and resulting in more drug going to the tumor and less affecting healthy tissue. Despite 30 years of research, however, specific targeting rarely succeeds in the therapeutic context.

Conventional antibody dependent chemotherapy (ADC) is designed with a toxic agent linked to a targeting antibody via a synthetic protease-cleavable linker. The efficacy of such ADC therapy is dependent on the ability of the target cell to bind to the antibody, the linker to be cleaved, and the uptake of the toxic agent into the target cell. Schrama, D. et al. (2006) Nature reviews. Drug discovery 5:147-159.

Antibody-targeted chemotherapy promised advantages over conventional therapy because it provides combinations of targeting ability, multiple cytotoxic agents, and improved therapeutic capacity with potentially less toxicity. Despite extensive research, clinically effective antibody-targeted chemotherapy remains elusive: major hurdles include the instability of the linkers between the antibody and chemotherapy drug, reduced tumor toxicity of the chemotherapeutic agent when bound to the antibody, and the inability of the conjugate to bind and enter tumor cells. In addition, these therapies did not allow for control over the size of the antibody-drug conjugates. WO 2015/191969 discloses treating lymphomas. WO 2014/055415 discloses cancer treatments. Nevala et al. (2016, Cancer Research, 76(13):3954-3964) discloses antibody-targeted chemotherapy for the treatment of Melanoma. Nevala et al. (2015, Cancer Immunology Research, 3: page B77) discloses targeted nano-immune conjugates to melanoma . WO 201 6/057554 discloses carrier-antibody compositions and methods of making and using the same.

There remains a need in the art for antibody-based cancer therapeutics that retain cytotoxic effect for targeted drug delivery to provide reliable and improved anti-tumor efficacy over prior therapeutics.

### SUMMARY OF THE INVENTION

Aspects and embodiments of the present invention are set forth in the claims. The references herein to methods of treatment are to be interpreted as references to the compounds, compositions and medicaments for use in methods of treatment of the human or animal body by therapy. Due to the unique aspects of the manufacturing of therapeutic antibodies and albumin-bound paclitaxel nanoparticles (also referred to as albumin-bound paclitaxel; e.g., ABRAXANE^{®}, ABX), humanized therapeutic monoclonal antibodies, including bevacizumab, trastuzumab and rituximab, bind with a high affinity to ABX, thereby offering the ability to specifically target the chemotherapeutic agent within ABX, paclitaxel, to the tumor. Furthermore, bevacizumab-coated ABX (AB160) was more efficacious than ABX alone in a mouse model of human melanoma.

However, it is contemplated that not all antibodies will bind to a desired degree to albumin (or ABX) to form nanoparticles. Therefore, one aspect of this disclosure relates to antibodies that do not contain an albumin-binding motif (e.g., do not complex with albumin and/or albumin-bound paclitaxel nanoparticles or do not complex to a desired degree) that are modified to contain one or more albumin-binding motifs as described herein. In another aspect, this disclosure relates to antibodies that do contain an albumin-binding motif and are modified to contain one or more additional albumin-binding motifs as described herein. Such antibodies include, but are not limited to, rituximab, trastuzimab, bevacizumab, and muromonab. In one example, this disclosure relates to methods of making modified antibodies that are modified to contain one or more albumin-binding motifs.

In another aspect, this disclosure relates to carrier proteins that do not contain an antibody-binding motif (e.g., do not complex with antibodies to form nanoparticle complexes or do not complex to the desired degree) that are modified to contain one or more antibody-binding motifs as described herein. In one aspect, this disclosure relates to carrier proteins (e.g., albumin) that do contain an antibody-binding motif and are modified to contain one or more additional antibody-binding motifs as described herein. In one example this disclosure relates to methods of making modified carrier proteins that are modified to contain one or more antibody-binding motifs.

This disclosure also relates to nanoparticle complexes containing the modified antibodies and/or carrier proteins as described herein, preferably including paclitaxel. In one example, aspects of the disclosure relate to methods of making the nanoparticle complexes. In another example, this disclosure relates to methods of using the nanoparticle complexes, e.g., to treat cancer.

Without being bound by theory, it is believed that paclitaxel interaction with the albumin increases the affinity of an antibody for albumin and allows formation of nanoparticle complexes. Nanoparticles of carrier proteins and antibodies, without paclitaxel, may be beneficial for treatment of cancer and other diseases. Accordingly, this description relates to complexes comprising modified carrier protein and/or antibodies (or other antigen binding agents, e.g., fusion proteins) without paclitaxel, with or without additional therapeutic agents. Without wishing to be bound by any theory, it is believed that addition of antibody-binding motifs to the carrier protein, and/or addition of albumin-binding motifs to the antibody (or binding agent) will increase the carrier protein-antibody binding to allow nanoparticle formation in the absence of paclitaxel.

In one example, this disclosure relates to a nanoparticle complex comprising a carrier protein having a modified polypeptide sequence that was modified to comprise at least one antibody-binding motif; antibodies, each antibody having an antigen-binding domain; and optionally a therapeutic agent; wherein the nanoparticle complex has binding specificity for the antigen *in vivo.*

In one example, this disclosure relates to a nanoparticle complex comprising albumin; an antibody or fusion protein having a modified polypeptide sequence that was modified to comprise at least one albumin-binding motif, said antibody or fusion protein having an antigen-binding domain; and optionally a therapeutic agent; wherein the nanoparticle complex has binding specificity for the antigen *in vivo.*

In one example, this disclosure relates to a nanoparticle complex comprising a carrier protein having a modified polypeptide sequence that was modified to comprise at least one antibody-binding motif; an antibody or fusion protein having a modified polypeptide sequence that was modified to comprise at least one albumin-binding motif, said antibody or fusion protein having an antigen-binding domain; and optionally a therapeutic agent; wherein the nanoparticle complex has binding specificity for the antigen *in vivo.*

In one example, this disclosure relates to a composition comprising nanoparticle complexes as described herein.

In one example, the antibody-binding motif comprises the amino acid sequence of SEQ ID NO.: 3, SEQ ID NO.:4, or SEQ ID NO.:5.

In one example, the albumin-binding motif comprises the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12.

In one embodiment, the therapeutic agent is paclitaxel.

In one embodiment, the complexes have an average size of less than 1 µm. In one embodiment, the nanoparticle complexes have an average size between about 90 nm and about 800 nm. In one embodiment, the nanoparticle complexes have an average size between about 90 nm and about 400 nm. In one embodiment, the nanoparticle complexes have an average size between about 90 nm and about 200 nm. In one embodiment, the nanoparticle complexes have an average size between about 100 nm and about 800 nm. In one embodiment, the nanoparticle complexes have an average size between about 100 nm and about 400 nm. In one embodiment, the nanoparticle complexes have an average size between about 100 nm and about 200 nm.

In one embodiment, the ratio of albumin-therapeutic agent to antibody is between 10:1 and 10:30. In one embodiment, each of the nanoparticle complexes comprises between about 100 and about 1000 antibodies. In one embodiment, each of the nanoparticle complexes comprises between about 100 and about 800 antibodies.

In one embodiment, the albumin is human serum albumin. In one embodiment, the human serum albumin is recombinant human serum albumin.

In one embodiment, the nanoparticle complex is lyophilized. In one embodiment, the composition comprising nanoparticle complexes is lyophilized. In one embodiment, the lyophilized nanoparticle complex or composition, upon reconstitution in an aqueous solution, comprises nanoparticle complexes that remain capable of binding to (recognize) the antigen *in vivo.*

In one embodiment, the carrier protein is albumin, ovalbumin, gelatin, elastin, an elastin-derived polypeptide, gliadin, legumin, zein, soy protein, milk protein, or whey protein. In one embodiment, the carrier protein is albumin. In one embodiment, the albumin is modified to contain additional antibody-binding motifs.

In one embodiment, the antibodies or fusion proteins are associated with the carrier protein through non-covalent bonds. In one embodiment, the antibodies or fusion proteins are associated with the carrier protein via the antibody-binding motif and/or the albumin-binding motif. In one embodiment, the paclitaxel is associated with the carrier protein through non-covalent bonds. In one embodiment, wherein the antibodies or fusion proteins are non-covalently associated with the carrier protein via the antibody-binding motif. In one embodiment, the antibodies or fusion proteins are non-covalently associated with the carrier protein via the albumin-binding motif.

In one embodiment, at least a subset of the modified carrier protein comprises more than one antibody-binding motif. In one embodiment, at least a subset of the modified antibodies or fusion proteins comprises more than one albumin-binding motif.

In one embodiment, the composition further comprises a pharmaceutically acceptable excipient.

In one aspect, this disclosure relates to a modified antibody having a modified polypeptide sequence that was modified to comprise at least one albumin-binding motif. In one example, the albumin-binding motif comprises the polypeptide sequence of SEQ ID NO.: 6, SEQ ID NO.: 7, SEQ ID NO.: 8, SEQ ID NO.: 9, SEQ ID NO.: 10, SEQ IDNO.: 11, or SEQ ID NO.: 12. In one embodiment, the modified antibody has a higher affinity for albumin than the antibody with an unmodified polypeptide sequence.

In one aspect, this disclosure relates to a modified carrier protein having a modified polypeptide sequence that was modified to comprise at least one antibody-binding motif. In one example, the antibody-binding motif comprises the polypeptide sequence of SEQ ID NO.: 3, SEQ ID NO.:4, or SEQ ID NO.:5. In one embodiment, the modified carrier protein has a higher affinity for an antibody than the carrier protein with an unmodified polypeptide sequence.

In one aspect, this disclosure relates to a method for making a nanoparticle complex, the method comprising combining albumin with an antibody having a modified polypeptide sequence that was modified to comprise at least one albumin-binding motif under conditions to form the nanoparticle complex. In one example, the carrier protein or the nanoparticle complex is combined with a therapeutic agent. In one example, the therapeutic agent is paclitaxel.

In one aspeetexample is disclosed a method for making a nanoparticle complex, the method comprising incubating a carrier protein having a modified polypeptide sequence that was modified to comprise at least one antibody-binding motif with an antibody. In one example, the carrier protein is albumin. In one example, the carrier protein or the nanoparticle complex is combined with a therapeutic agent. In one example, the therapeutic agent is paclitaxel. In one example, the antibody is a modified antibody having a modified polypeptide sequence that was modified to comprise at least one albumin-binding motif.

In one example, the antibody-binding motif comprises the polypeptide sequence of SEQ ID NO.: 3, SEQ ID NO.:4, or SEQ ID NO.:5. In one example, the albumin-binding motif comprises the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12.

In one aspeetexample is disclosed a method for making a modified antibody, said method comprising providing an antibody having a polypeptide sequence and modifying the polypeptide sequence to comprise an albumin-binding motif, wherein the modified antibody has a higher affinity for binding to albumin than the antibody before modification. In one example, the albumin-binding motif comprises the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12. In one example, the polypeptide sequence did not comprise the albumin-binding motif prior to modification.

In one example is disclosed a method for making a modified carrier protein, said method comprising providing a carrier protein having a polypeptide sequence and modifying the polypeptide sequence to comprise an antibody-binding motif, wherein the modified carrier protein has a higher affinity for binding to an antibody than the carrier protein before modification. In one example, the antibody-binding motif comprises the amino acid sequence of SEQ ID NO.: 3, SEQ ID NO.:4, or SEQ ID NO.:5. In one example, the carrier protein is albumin, ovalbumin, gelatin, elastin, an elastin-derived polypeptide, gliadin, legumin, zein, soy protein, milk protein, or whey protein. In one example, the polypeptide sequence did not comprise the antibody-binding motif prior to modification.

In one example is disclosed a method for treating a cancer in a patient, the method comprising administering to the patient a therapeutically effective amount of the nanoparticle complexes comprising a modified antibody and/or modified carrier protein as described herein, wherein the cancer expresses the antigen. In one example, the composition is administered intravenously. In one example, the composition is administered via direct injection or perfusion into a tumor.

### BRIEF DESCRIPTION OF THE DRAWINGS

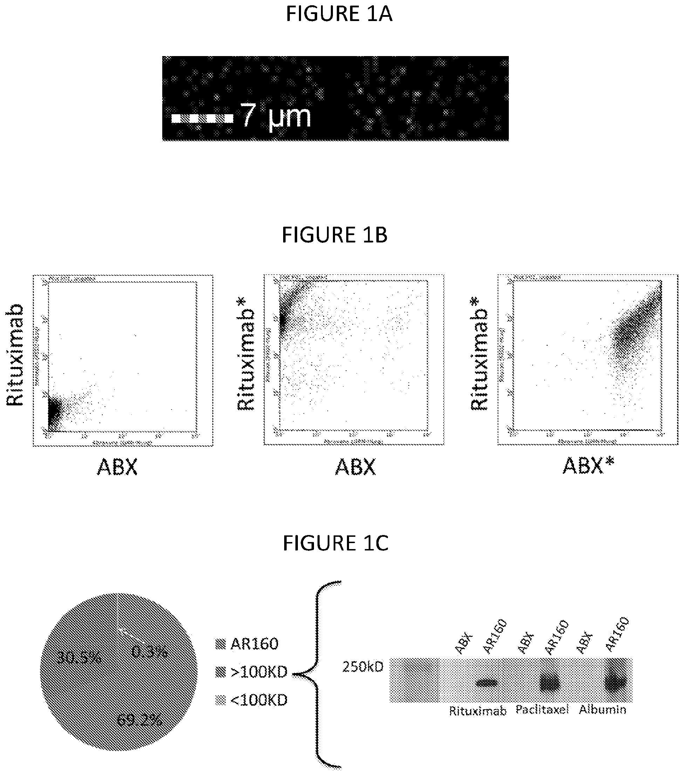

FIG. 1A shows an ImageStream^{®} image of nanoparticles formed after co-incubation of fluorescently labeled rituximab (green) with ABRAXANE^{®}.
FIG. 1B shows flow cytometric analyses of AR160 made with unlabeled rituximab and unlabeled ABRAXANE^{®} (ABX) (left panel); fluorescently labeled (*) rituximab and unlabeled ABX (middle panel); or fluorescently labeled rituximab and fluorescently labeled ABX (right panel).
FIG. 1C is a graphical representation of the amount of paclitaxel present in each fraction of AR160 after separation into particulate fraction (AR160; blue), proteins larger than 100 kilodaltons (KD) (>100 KD; red), and proteins smaller than 100 KD (< 100 KD; green). Paclitaxel concentration in each fraction was determined by HPLC and showed about 69.2% of paclitaxel is in the particulate and the remaining paclitaxel is among proteins greater the 100kD (30.5%). Western blot was performed on the greater than 100kD fraction and rituximab, paclitaxel and albumin co-localized in a band of approximately 200kD.

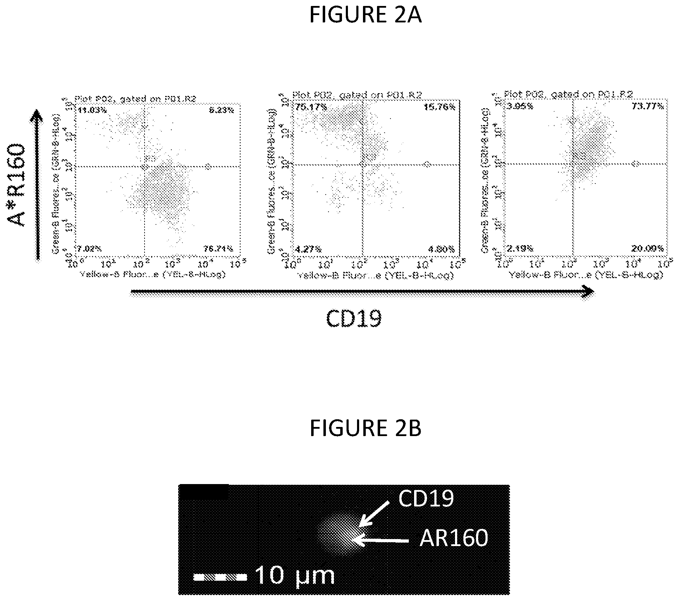

FIG. 2A shows flow cytometric analysis of Daudi cells incubated with PE anti-human CD19 (left panel), fluorescent AR160 (middle panel), or both (right panel). Daudi cells were about 75% positive for CD19, AR160 or both. FIG. 2B shows an ImageStream image of a Daudi cell from the same experiment as FIG. 2A, labeled with both CD19 (red) and fluorescent AR160 (green).

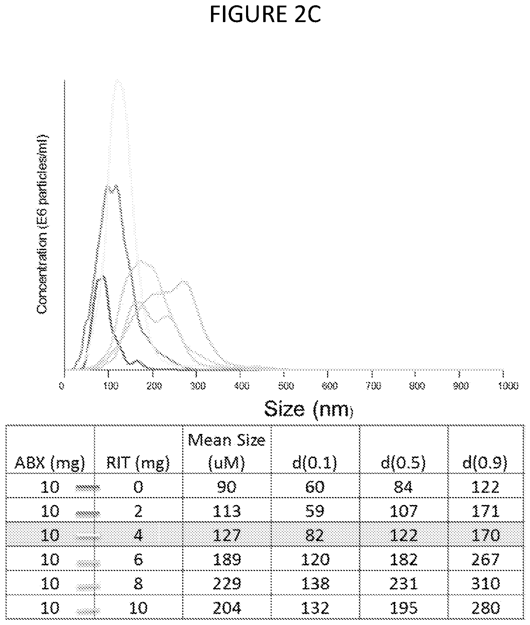

FIG. 2C is a graphical representation of particle concentrations of each size when 10 mg ABX is incubated with the indicated amount of rituximab (RIT) and analyzed for size distribution by NanoSight (Malvern Instruments Ltd, Worcestershire, UK). The table shows the size distributions (mean, 10^{th} percentile, 50^{th} percentile, and 90^{th} percentile).

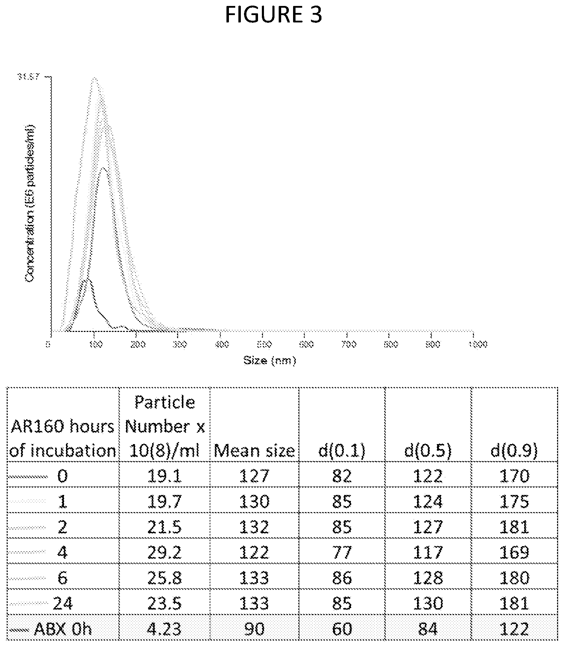

FIG. 3 is a graphical representation of *in vitro* stability of AR160 in normal saline for intravenous infusion. Clinical grade preps of AR160 were exposed at room temperature for different durations (0h, 1h, 2h, 4h, 6h and 24h) in normal saline. At the end of each incubation, NanoSight analyses of the particles were performed to assess particle numbers as well as their size distributions (mean, 10^{th} percentile, 50^{th} percentile, and 90^{th} percentile). ABX alone was analyzed at each time point as a control.

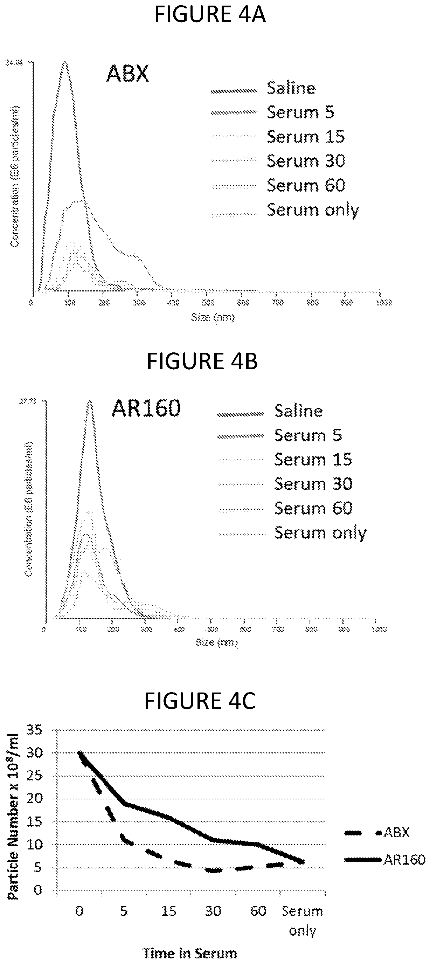

FIG. 4A is a graphical representation of the stability of ABX in human AB serum. 30×10⁸ particles were added to human AB serum and incubated for 60 minutes. Particle size and numbers were determined at 5, 15, 30, and 60 minutes after being added to the AB serum. ABX in saline was used as control.
FIG. 4B is a graphical representation of the stability of AR160 in human AB serum. 30×10⁸ particles were added to human AB serum and incubated for 60 minutes. Particle size and numbers were determined at 5, 15, 30, and 60 minutes after being added to the AB serum. ABX in saline was used as control.
FIG. 4C is a graphical representation of the particle number of ABX (dotted line) relative to AR160 (solid line) after incubation in AB serum.

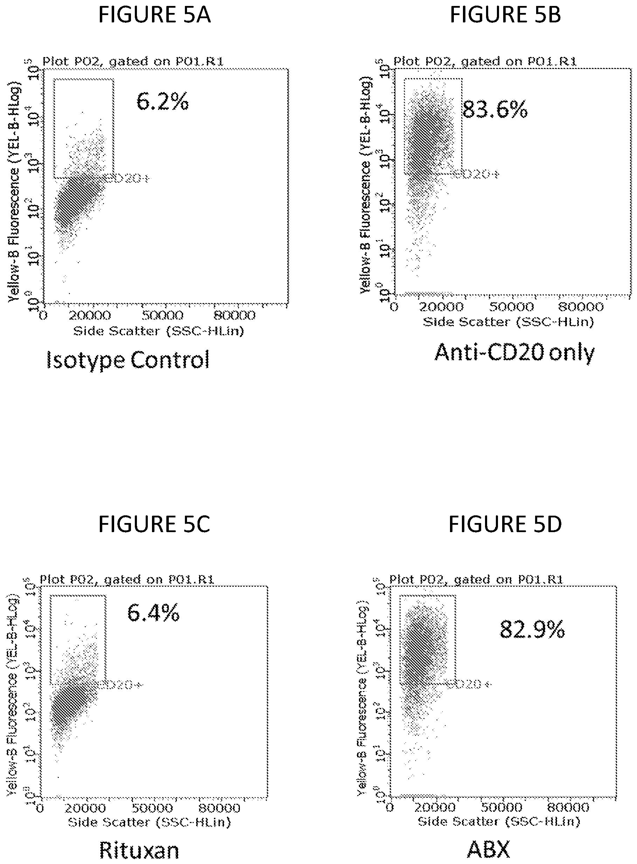

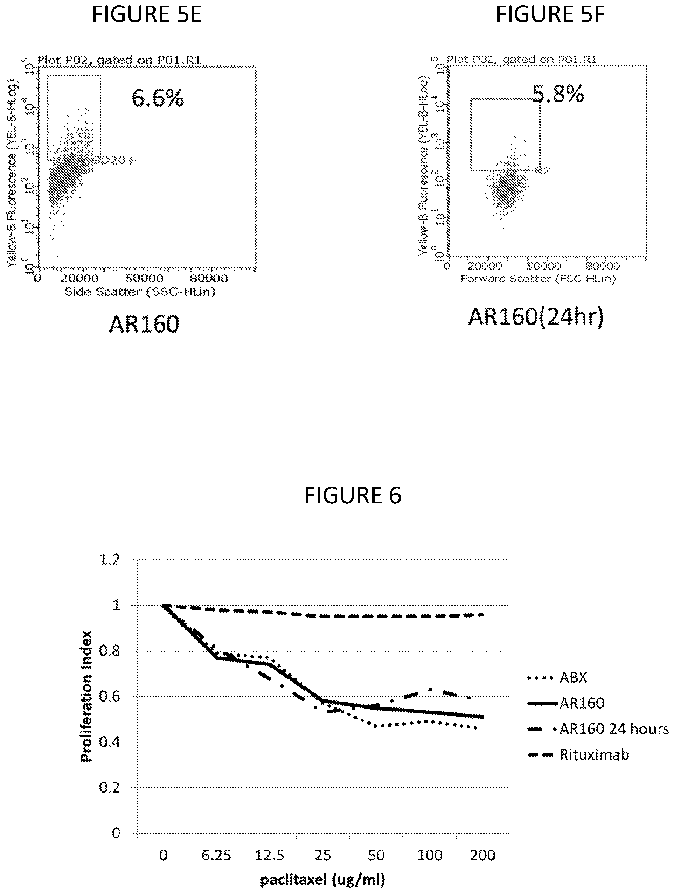

FIGs. 5A-5F represent flow cytometric analysis of Daudi cells pre-treated with isotype control antibody (FIG. 5A), no treatment (FIG. 5B), rituximab (FIG. 5C), ABRAXANE^{®} (FIG. 5D), AR160 (FIG. 5E), or AR160 that was incubated in saline for 24 hours (FIG. 5F), then labeled with fluorescently-labeled anti-human CD20 antibody.
FIG. 6 is a graphical representation of the level of proliferation of Daudi cells after overnight treatment with EdU and ABX, AR160, AR160 that was incubated in saline for 24 hours (AR160 24 hours), or rituximab, at the indicated paclitaxel concentrations. The level of proliferation was determined by staining cells with FITC labeled anti-EdU. The proliferation index was calculated by normalization to the untreated positive control.

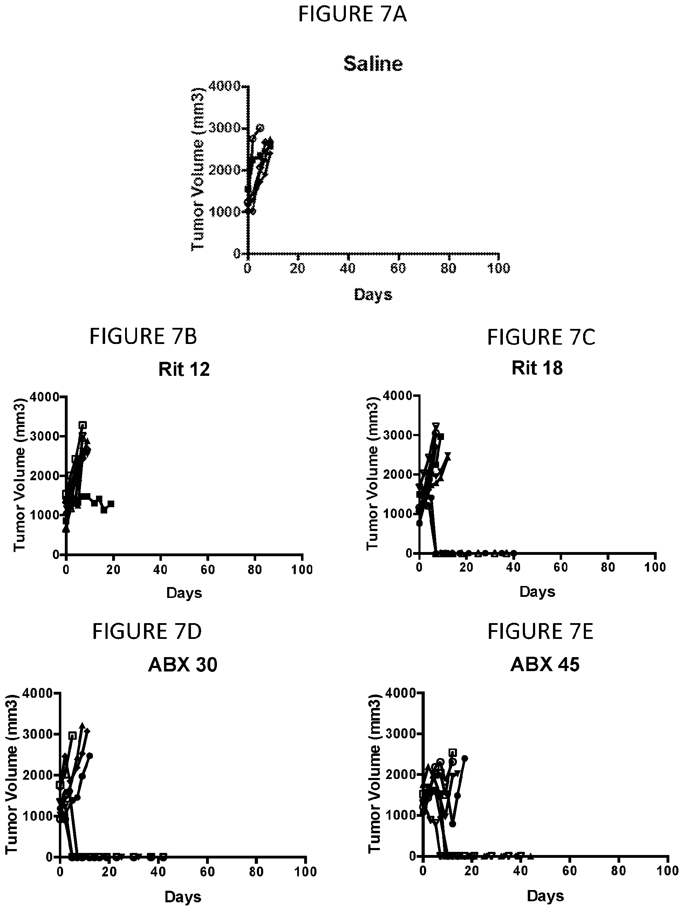

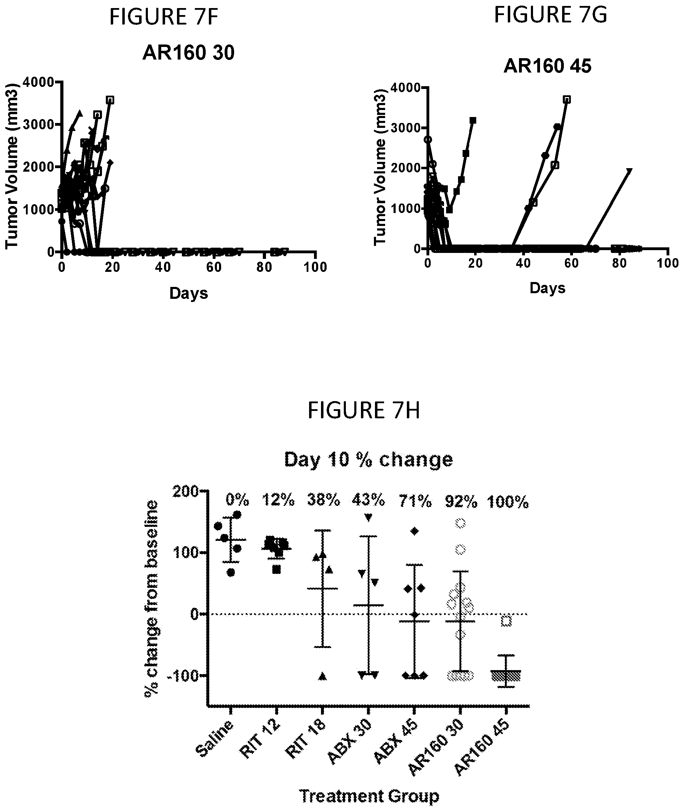

FIGs. 7A-7G represent tumor volume over time in mice treated with saline (FIG. 7A), rituximab at 12 mg/kg (FIG. 7B) or 18 mg/kg (FIG. 7C), ABRAXANE^{®} at 30 mg/kg (FIG. 7D) or 45 mg/kg (FIG. 7E), or AR160 at 30 mg/kg (FIG. 7F) or 45 mg/kg (FIG. 7G). Dosages for ABX and AR160 are based on paclitaxel. FIG. 7H represents the percent change in baseline tumor volume, based on the data from FIGs. 7A-7G.

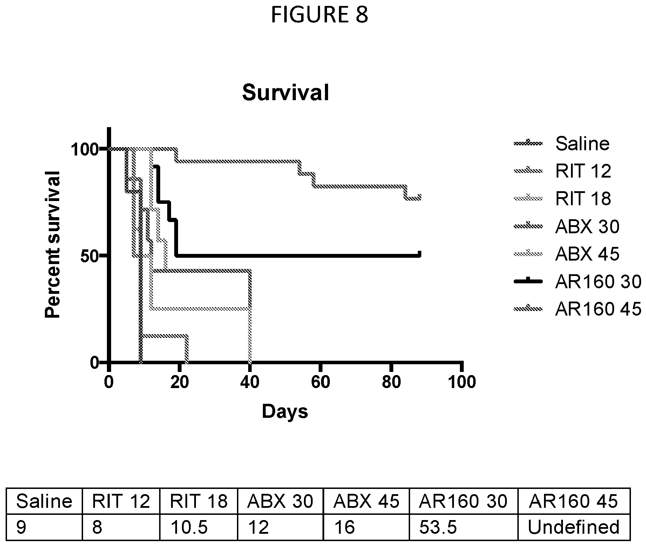

FIG. 8 represents a Kaplan-Meier curve showing survival of the mice from the experiment represented in FIGs. 7A-7H. The table provides median survival (days) for mice in each cohort.

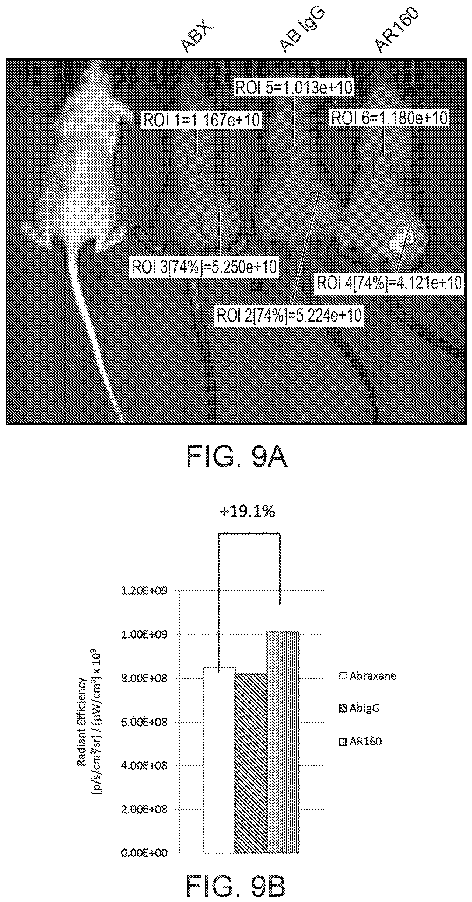

FIG. 9A is a photograph with IVIS Spectrum fluorescent overlay to show drug deposition in the tumors of mice treated with fluorescently-labeled ABX alone, ABX with control antibody (AB IgG), or AR160. ABX was labeled with AlexaFluor 750, then bound to either to IVIG as a negative control or rituximab (AR160) and IVIS Spectrum (Perkin Elmer) was employed to fluorescently quantify the concentration of ABX in each tumor. FIG. 9B is a graphical representation of fluorescence from the tumors of the animals shown in FIG. 9A. Regions of interest (ROI) were made for the tumor (irregular defined area in FIG. 9A) and a distal area on the back (circle in FIG. 9A) of each mouse as background. The background ROI for each mouse was subtracted from the tumor ROI, and the resultant radiant efficiencies were graphed.

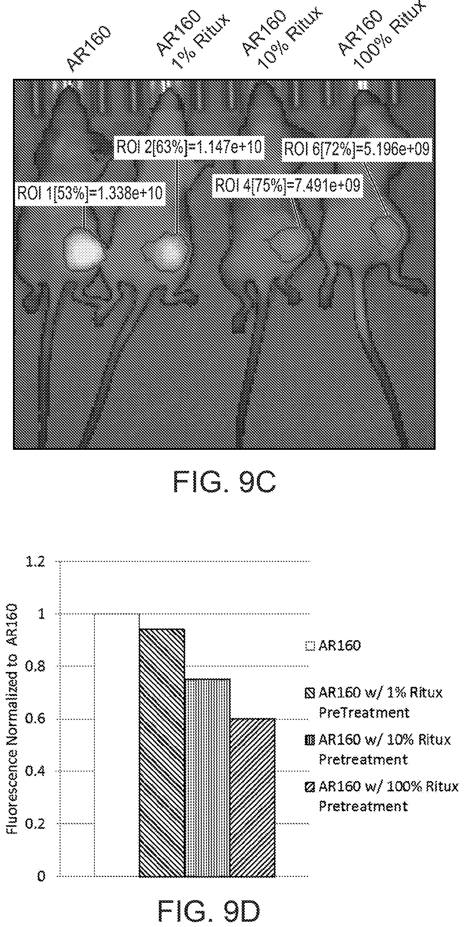

FIG. 9C is a photograph with IVIS Spectrum fluorescent overlay to show drug deposition in the tumors of mice pre-treated with 1%, 10% or 100% dose of rituximab 24 hours before treatment with AR160. FIG. 9D is a graphical representation of fluorescence from the tumors of the animals shown in FIG. 9C. Regions of interest (ROI) were made for the tumor (irregular defined area in FIG. 9C) and a distal area on the back (circle in FIG. 9A) of each mouse as background. The background ROI for each mouse was subtracted from the tumor ROI, and the resultant radiant efficiencies were graphed.

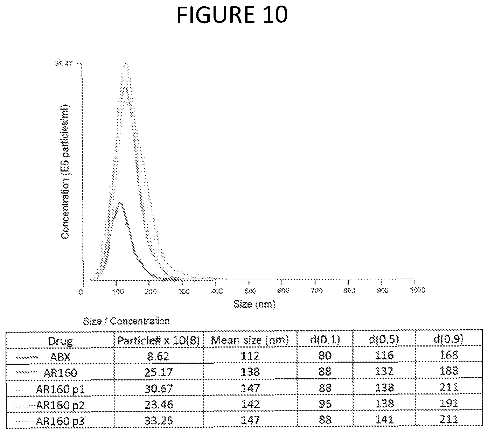

FIG. 10 is a graphical representation of size analysis of AR160 made in the laboratory (AR160) versus three batches made in the pharmacy (AR160 p1, p2, or p3). Sizes (diameters in nm) are represented as mean size, 10th percentile, d(0.1); 50th percentile, d(0.5); and 90th percentile, d(0.9). The number of particles in each prep was also determined (×10⁸/mL).

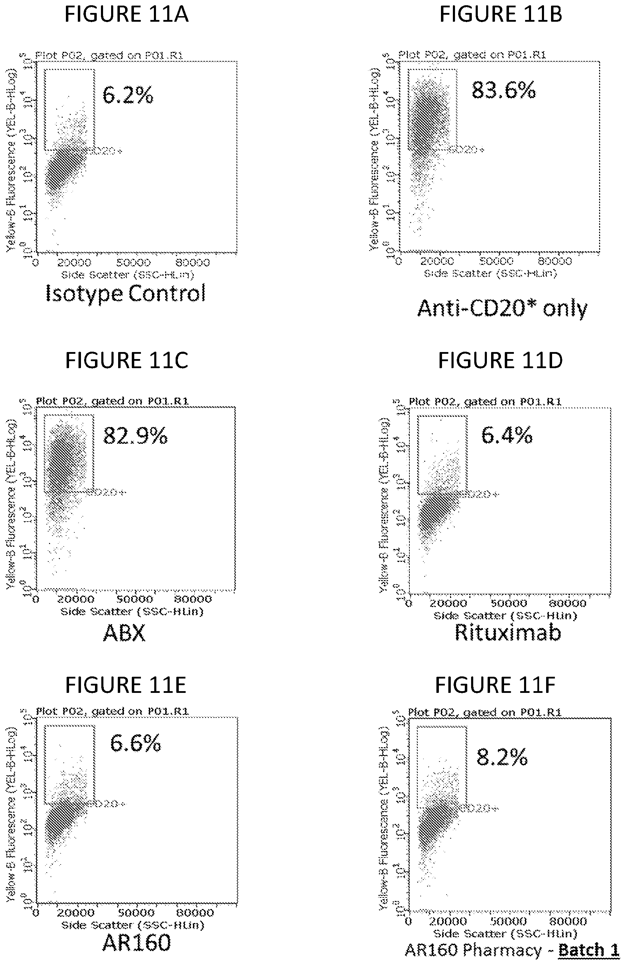

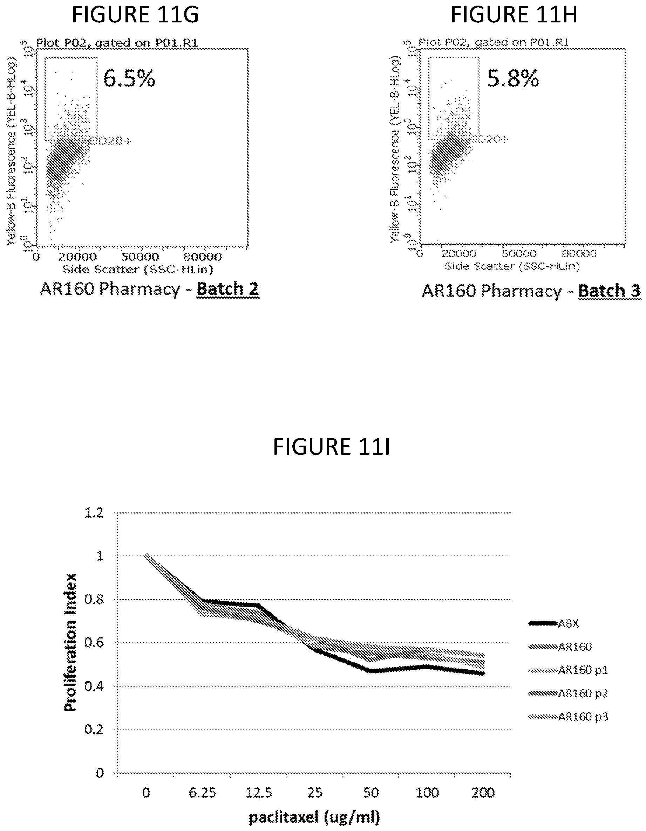

FIGs. 11A-11H represent flow cytometric analysis of Daudi cells pre-treated with isotype control antibody (FIG. 11A), no pre-treatment (FIG. 11B), ABRAXANE^{®} (FIG. 11C), rituximab (FIG. 11D), lab-made AR160 (FIG. 11E), or each of the three pharmacy-made AR160 batches (FIGs. 11F-11H), then labeled with fluorescently-labeled anti-human CD20 antibody.
FIG. 11I is a graphical representation of the level of proliferation of Daudi cells after overnight treatment with EdU and ABX, AR160, or each of the three pharmacy-made AR160 batches, at the indicated paclitaxel concentrations. The level of proliferation was determined by staining cells with FITC labeled anti-EdU. The proliferation index was calculated by normalization to the untreated positive control.

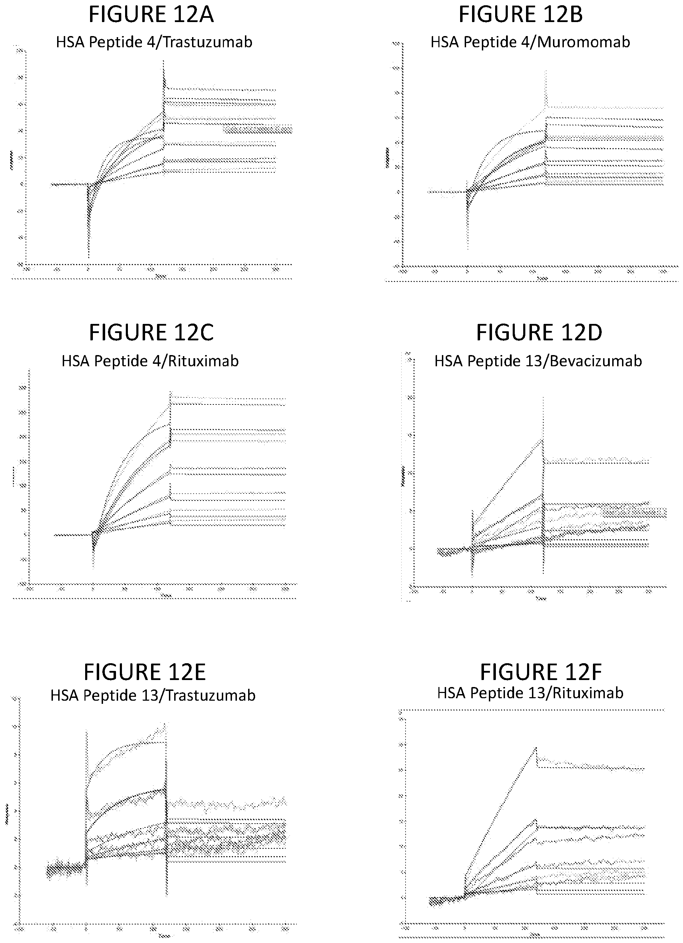

FIG. 12A represents binding of trastuzumab (HERCEPTIN^{®}) to HSA Peptide 4 (SEQ ID NO.: 3). FIG. 12B represents binding of muromonab (also called muromonab-CD3 or AKT3; ORTHOCLONE OKT3^{®}) to HSA Peptide 4. FIG. 12C represents binding of rituximab (RITUXAN^{®}) to HSA Peptide 4.
FIG. 12D represents binding of bevacizumab (AVASTIN^{®}) to HSA peptide 13 (SEQ ID NO.: 4). FIG. 12E represents binding of trastuzumab to HSA peptide 13. FIG. 12F represents binding of rituximab to HSA Peptide 13.

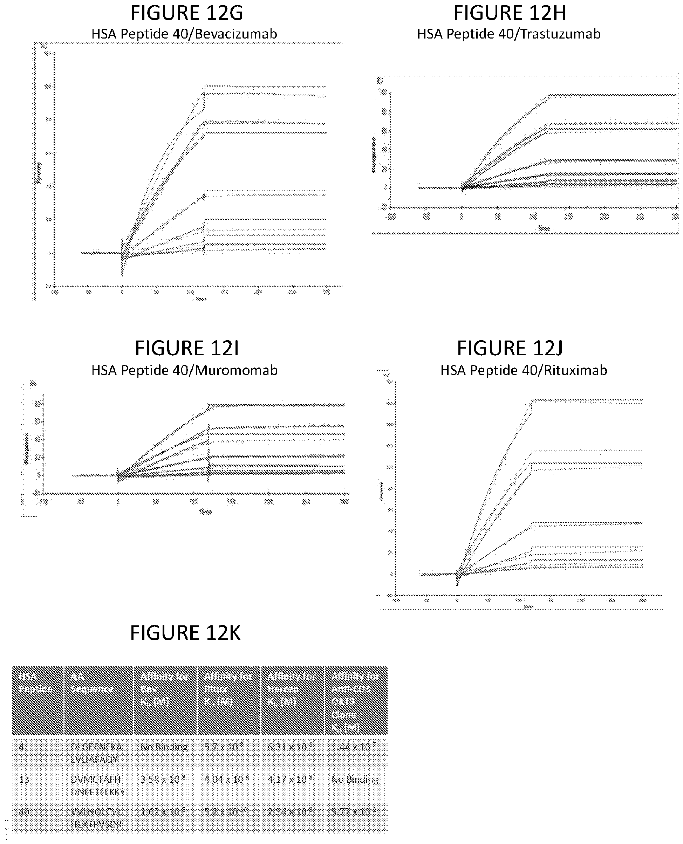

FIG. 12G represents binding of bevacizumab to HSA peptide 40 (HSA amino acids 455-472; SEQ ID NO.: 5). FIG. 12H represents binding of trastuzumab to HSA peptide 40. FIG. 12I represents binding of muromonab to HSA peptide 40. FIG. 12J represents binding of rituximab to HSA peptide 40.
FIG. 12K provides the amino acid sequences of each HSA peptide, as well as the affinity of each antibody for each HSA peptide.

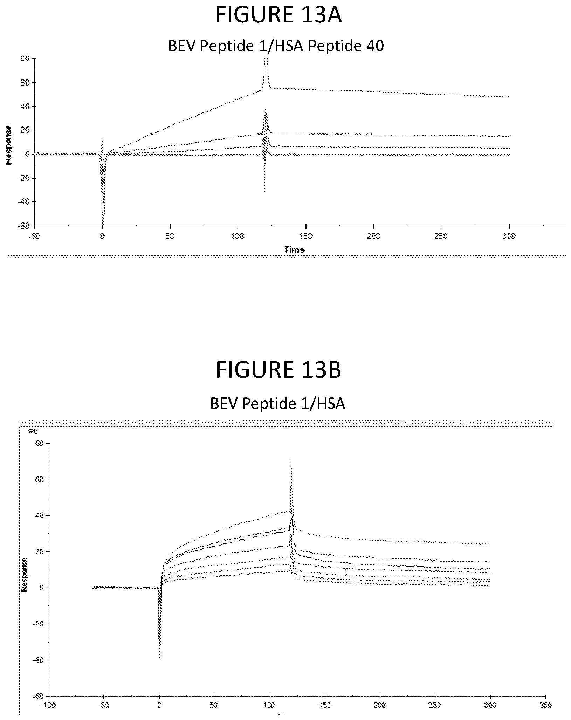

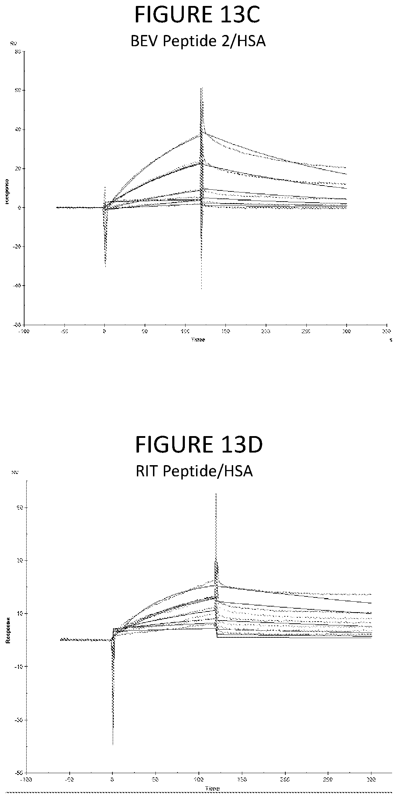

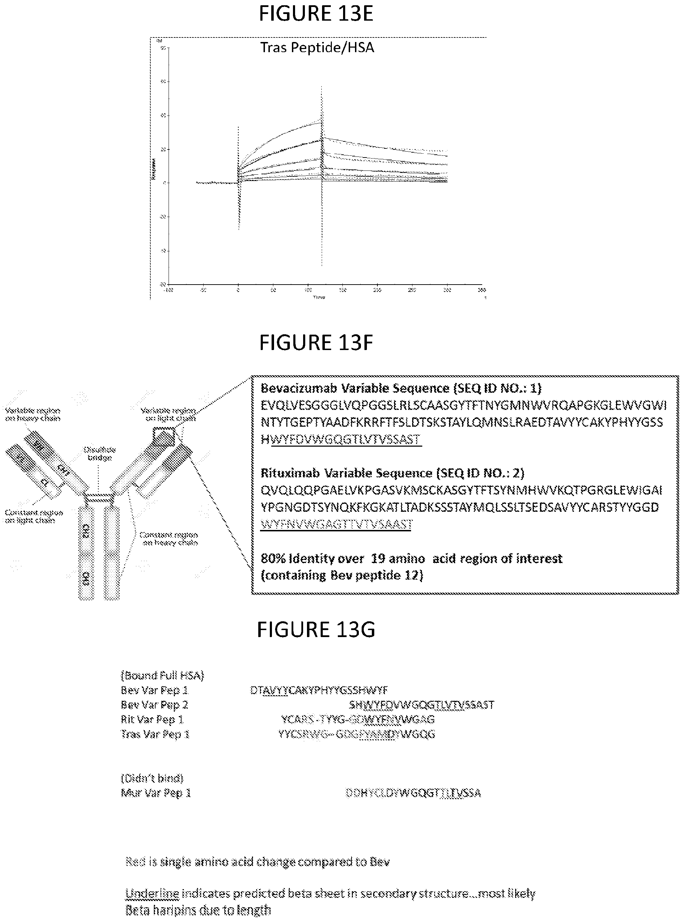

FIG. 13A represents binding of a bevacizumab peptide (BEV Peptide 1; amino acids 111-125; SEQ ID NO.: 7) to HSA peptide 40. FIG. 13B represents binding of bevacizumab variable peptide 1 (SEQ ID NO.: 7) to HSA. FIG. 13C represents binding of bevacizumab variable peptide 2 (SEQ ID NO.: 6) to HSA. FIG. 13D represents binding of rituximab variable peptide 1 (SEQ ID NO.: 9) to HSA. FIG. 13E represents binding of trastuzumab variable peptide 1 (SEQ ID NO.: 11) to HSA.
FIG. 13F is a drawing representing an antibody. The small blue box indicates the approximate position on bevacizumab and rituximab that bind to albumin in albumin-paclitaxel complexes to form nanoparticles. The large box provides the variable sequences for bevacizumab (SEQ ID NO.: 1) and rituximab (SEQ ID NO.: 2). The albumin-binding sequences of each are underlined and shown in blue (bevacizumab; SEQ ID NO.: 8) and red (rituximab; SEQ ID NO.: 10). FIG. 13G provides the sequences of several of the peptides used in FIGs. 13A-13E, including analysis of single amino acid changes compared to bevacizumab (in red) and predicted beta sheet structures (underlined).

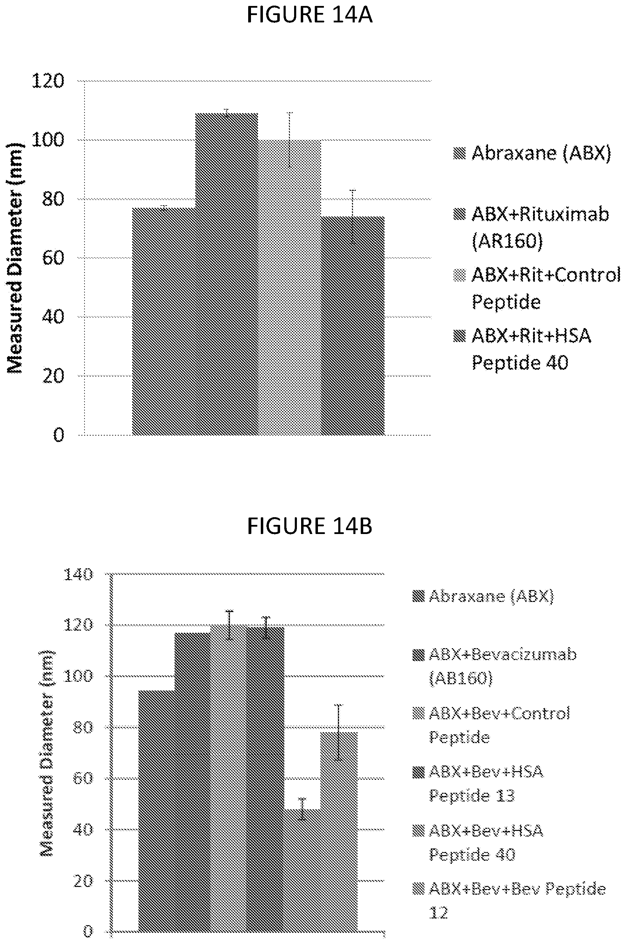

FIG. 14A represents the effect of HSA Peptide 40 competition on nanoparticle formation with rituximab. ABX (5 mg/mL) was incubated with rituximab (2 mg/mL) and either no peptide (red bar), a control peptide (HSA Peptide 10; green bar), or HSA Peptide 40 (purple bar). Peptides were added at a 10-fold molar excess compared to antibody. Diameter was measured using a Malvern Nanosizer at a 1:200 dilution.
FIG. 14B represents the effect of HSA Peptide 40 competition on nanoparticle formation with bevacizumab. ABX (10 mg/mL) was incubated with bevacizumab (4 mg/mL) and either no peptide (red bar), a control peptide (HSA Peptide 10; green bar), HSA Peptide 13 (purple bar), HSA Peptide 40 (blue bar), or BEV Peptide 12 (orange bar). Peptides were added at a 10-fold molar excess compared to antibody. Diameter was measured using a Malvern Nanosizer at a 1:200 dilution.

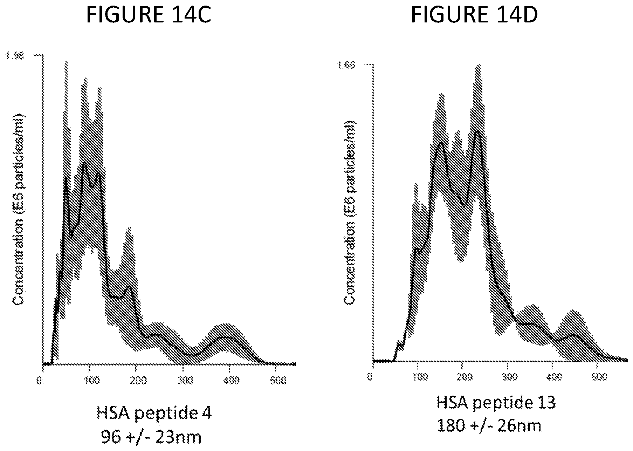

FIG. 14C shows the effect of HSA peptide 4 competition on complex formation between rituximab and ABX. The resulting average particle size was 96 nm (+/- 23 nm). FIG. 14D shows the effect of HSA peptide 13 competition on complex formation between rituximab and ABX. The resulting average particle size was 180 nm (+/- 26 nm).

### DETAILED DESCRIPTION

After reading this description it will become apparent to one skilled in the art how to implement the invention in various alternative embodiments and alternative applications. However, all the various embodiments of the present invention will not be described herein.

The detailed description of the invention is divided into various sections only for the reader's convenience and disclosure found in any section may be combined with that in another section. Titles or subtitles may be used in the specification for the convenience of a reader, which are not intended to influence the scope of the present invention.

### Definitions

Unless defined otherwise, all technical and scientific terms used herein have the same meaning as commonly understood by one of ordinary skill in the art to which this invention belongs. In this specification and in the claims that follow, reference will be made to a number of terms that shall be defined to have the following meanings:

As used herein, the singular forms "a", "an" and "the" are intended to include the plural forms as well, unless the context clearly indicates otherwise.

"Optional" or "optionally" means that the subsequently described event or circumstance can or cannot occur, and that the description includes instances where the event or circumstance occurs and instances where it does not.

The term "about" when used before a numerical designation, e.g., temperature, time, amount, concentration, and such other, including a range, indicates approximations which may vary by ( + ) or ( - ) 10%, 5%,1%, or any subrange or subvalue there between. Preferably, the term "about" when used with regard to a dose amount means that the dose may vary by +/- 10%.

"Comprising" or "comprises" is intended to mean that the compositions and methods include the recited elements, but not excluding others. "Consisting essentially of' when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of' shall mean excluding more than trace elements of other ingredients and substantial method steps. Embodiments defined by each of these transition terms are within the scope of this invention.

The term "nanoparticle" as used herein refers to particles with at least one dimension less than 5 microns. In preferred embodiments, such as for intravenous administration, the nanoparticle is less than 1 micron. For direct administration, the nanoparticle is larger. Even larger particles are expressly contemplated by the invention.

In a population of particles, the size of individual particles is distributed about a mean. Particle sizes for the population can therefore be represented by an average, and also by percentiles. D50 is the particle size below which 50% of the particles fall. 10% of particles are smaller than the D10 value and 90% of particles are smaller than D90. Where unclear, the "average" size is equivalent to D50.

The term "carrier protein" as used herein refers to proteins that function to transport antibodies or fusion proteins. The antibodies or fusion proteins of the present disclosure can reversibly bind to the carrier proteins. Examples of carrier proteins are discussed in more detail below.

The term "core" as used herein refers to central or inner portion of the nanoparticle which may be comprised of a carrier protein, a carrier protein and a therapeutic agent, or other agents or combination of agents.

The term "buffer" encompasses those agents which maintain the solution pH in an acceptable range prior to lyophilization and may include succinate (sodium or potassium), histidine, phosphate (sodium or potassium), Tris (tris(hydroxymethyl)aminomethane), diethanolamine, citrate (sodium). In one example, buffer of this disclosure has a pH in the range from about 5.5 to about 6.5; and preferably has a pH of about 6.0. Examples of buffers that will control the pH in this range include succinate (such as sodium succinate), gluconate, histidine, citrate and other organic acid buffers.

The term "pharmaceutical formulation" refers to preparations which are in such form as to permit the active ingredients to be effective, and which contains no additional components which are toxic to the subjects to which the formulation would be administered.

"Pharmaceutically acceptable" excipients (vehicles, additives) are those which can reasonably be administered to a subject mammal to provide an effective dose of the active ingredient employed.

The term "therapeutic agent" as used herein means an agent which is therapeutically useful, e.g., an agent for the treatment, remission or attenuation of a disease state, physiological condition, symptoms, or etiological factors, or for the evaluation or diagnosis thereof. A therapeutic agent may include a radioisotope or other molecule for visualization; or an anti-cancer agent, e.g. a chemotherapy or radiotherapy agent. In some examples, one or more therapeutic agents or types of agents may be expressly excluded from the composition.

The term "antibody" or "antibodies" as used herein refers to immunoglobulin molecules and immunologically active portions of immunoglobulin molecules (i.e., molecules that contain an antigen binding site that immuno-specifically bind an antigen). The term also refers to antibodies comprised of two immunoglobulin heavy chains and two immunoglobulin light chains as well as a variety of forms including full length antibodies and portions thereof; including, for example, an immunoglobulin molecule, a monoclonal antibody, a chimeric antibody, a CDR-grafted antibody, a humanized antibody, a Fab, a Fab', a F(ab')2, a Fv, a disulfide linked Fv, a scFv, a single domain antibody (dAb), a diabody, a multispecific antibody, a dual specific antibody, an anti-idiotypic antibody, a bispecific antibody, a functionally active epitope-binding fragment thereof, bifunctional hybrid antibodies (e.g., Lanzavecchia et al., Eur. J. Immunol. 17, 105 (1987)) and single chains (e.g., Huston et al., Proc. Natl. Acad. Sci. U.S.A., 85, 5879-5883 (1988) and Bird et al., Science 242, 423-426 (1988). (See, generally, Hood et al., Immunology, Benjamin, N.Y., 2ND ed. (1984); Harlow and Lane, Antibodies. A Laboratory Manual, Cold Spring Harbor Laboratory (1988); Hunkapiller and Hood, Nature, 323, 15-16 (1986). The antibody may be of any type (e.g., IgG, IgA, IgM, IgE or IgD). Preferably, the antibody is IgG. An antibody may be non-human (e.g., from mouse, goat, or any other animal), fully human, humanized, or chimeric.

The term "fusion protein" as used herein refers to a protein containing at least two domains, where one domain is derived from one protein and the other domain is derived from a different protein. Aflibercept (ZALTRAP^{®}) is a fusion protein that has been approved by the FDA for treatment of colorectal cancer. Other fusion proteins for the treatment of cancer are known, including trebanabib (AMG386, Amgen) and APG 101 (APOCEPT, Apogenix). In some embodiments, the fusion protein is a Fc-fusion protein.

The terms "lyophilized," and "lyophilization" as used herein refer to a process by which the material (e.g., nanoparticles) to be dried is first frozen and then the ice or frozen solvent is removed by sublimation in a vacuum environment. An excipient may be included in pre-lyophilized formulations to enhance stability of the lyophilized product upon storage. In some embodiments, the carrier protein, antibodies or fusion proteins, and/or therapeutic agent are lyophilized separately. In other embodiments, the carrier protein, antibodies or fusion proteins, and/or therapeutic agent are first combined and then lyophilized. The lyophilized sample may further contain additional excipients. Lyophilization of antibody-albumin-paclitaxel nanoparticle complexes is described, for example, in U.S. 9,446,148.

The term "treating" or "treatment" covers the treatment of a disease or disorder (e.g., cancer), in a subject, such as a human, and includes: (i) inhibiting a disease or disorder, i.e., arresting its development; (ii) relieving a disease or disorder, i.e., causing regression of the disease or disorder; (iii) slowing progression of the disease or disorder; and/or (iv) inhibiting, relieving, or slowing progression of one or more symptoms of the disease or disorder.

The term "kill" with respect to a cell/cell population is directed to include any type of manipulation that will lead to the death of that cell/cell population.

As used herein, the term "outer surface" when referring to the nanoparticle core refers to a portion of the core which is on the outside of the core. In some embodiments, the binding agents, e.g. antibodies or fusion proteins, are associated with the outer surface.

As used herein, the phrase "while retaining antibody specificity" indicates that the antibodies associated with albumin continue to recognize (bind to) the epitope being targeted. Antibody specificity may be retained in the context of the large nanoparticle and/or after dissociation or partial dissociation of the nanoparticle *in vivo.*

As used herein, the term "identity" or "similarity" refers to sequence similarity between two peptides or between two nucleic acid molecules. "Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

A polynucleotide or polynucleotide region (or a polypeptide or polypeptide region) has a certain percentage (for example, 60 %, 65 %, 70 %, 75 %, 80 %, 85 %, 90 %, 95 %, 98 % or 99 %) of "sequence identity" or "similarity" to another sequence means that, when aligned, that percentage of bases (or amino acids) are the same in comparing the two sequences. This alignment and the percent sequence identity can be determined using software programs known in the art, for example those described in Ausubel et al. eds. (2007) Current Protocols in Molecular Biology. Biologically equivalent polynucleotides are those having the above-noted specified percent sequence identity and encoding a polypeptide having the same or similar biological activity.

The term "modified" when referring to the modified carrier proteins or modified binding agents (e.g., antibodies) described herein can indicate that the indicated polypeptide sequence is added to the protein (or other molecule). For example, a modified antibody may be an antibody that has been modified (engineered, altered, mutated) to contain an albumin-binding motif that was not present in the unmodified antibody, or modified to contain an albumin-binding motif in addition to one that was already present. In one embodiment, the motifs may be added by changing (mutating) amino acids present in the protein. In one embodiment, the motifs may be added by insertion of the motif sequence into the protein (or other molecule). In one embodiment, the motifs may be added by covalently binding the motif sequence to the protein (or other molecule).

Additionally, some terms used in this specification are more specifically defined below.

### Modified Binding Agents

In one aspect, this disclosure relates to a modified antibody having a modified polypeptide sequence that was modified to comprise at least one albumin-binding motif. In one example, the albumin-binding motif comprises the polypeptide sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12. In one embodiment, the modified antibody binds albumin (is capable of binding albumin). In one embodiment, the modified antibody has a higher affinity for albumin than the antibody with an unmodified polypeptide sequence.

In one aspect is disclosed a method for making a modified antibody, said method comprising providing an antibody having a polypeptide sequence and modifying the polypeptide sequence to comprise an albumin-binding motif. In one example, the modified antibody has a higher affinity for binding to albumin than the antibody before modification. In one example, the albumin-binding motif comprises the amino acid sequence of SEQ ID NO.: 6, SEQ ID NO.: 7, SEQ ID NO.: 8, SEQ ID NO.: 9, SEQ ID NO.:10, SEQ ID NO.: 11, or SEQ ID NO.: 12. In one example, the polypeptide sequence did not comprise the albumin-binding motif prior to modification.

In one example, the albumin-binding motif comprises the polypeptide sequence of SEQ ID NO.: 6. In one example, the albumin-binding motif comprises a polypeptide sequence having at least 70% sequence identity to the polypeptide sequence of SEQ ID NO.: 6. In one example, the albumin-binding motif comprises a polypeptide sequence having at least 80% sequence identity to the polypeptide sequence of SEQ ID NO.: 6. In one example, the albumin-binding motif comprises a polypeptide sequence having at least 85% sequence identity to the polypeptide sequence of SEQ ID NO.: 6. In one example, the albumin-binding motif comprises a polypeptide sequence having at least 90% sequence identity to the polypeptide sequence of SEQ ID NO.: 6. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 6. In one example, the albumin-binding motif comprises a truncated polypeptide sequence of SEQ ID NO. 6 comprising at least 10, 11, 12, 13, 14, 15, 16, 17, 18, 19 or 20 amino acid residues.

In one example, the albumin-binding motif comprises the polypeptide sequence of SEQ ID NO.: 7. In one example, the albumin-binding motif comprises a polypeptide sequence having at least 70% sequence identity to the polypeptide sequence of SEQ ID NO.: 7. In one example, the albumin-binding motif comprises a polypeptide sequence having at least 80% sequence identity to the polypeptide sequence of SEQ ID NO.: 7. In one example, the albumin-binding motif comprises a polypeptide sequence having at least 85% sequence identity to the polypeptide sequence of SEQ ID NO.: 7. In one example, the albumin-binding motif comprises a polypeptide sequence having at least 90% sequence identity to the polypeptide sequence of SEQ ID NO.: 7. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 7. In one example, the albumin-binding motif comprises a truncated polypeptide sequence of SEQ ID NO. 7 comprising at least 10, 11, 12, 13, 14, 15, 16, 17, 18, 19 or 20 amino acid residues.

In one example, the albumin-binding motif comprises the polypeptide sequence of SEQ ID NO.: 8. In one example, the albumin-binding motif comprises a polypeptide sequence having at least 70% sequence identity to the polypeptide sequence of SEQ ID NO.: 8. In one example, the albumin-binding motif comprises a polypeptide sequence having at least 80% sequence identity to the polypeptide sequence of SEQ ID NO.: 8. In one example, the albumin-binding motif comprises a polypeptide sequence having at least 85% sequence identity to the polypeptide sequence of SEQ ID NO.: 8. In one example, the albumin-binding motif comprises a polypeptide sequence having at least 90% sequence identity to the polypeptide sequence of SEQ ID NO.: 8. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 8. In one example, the albumin-binding motif comprises a truncated polypeptide sequence of SEQ ID NO. 8 comprising at least 10, 11, 12, 13, 14, 15, 16, 17, or 18 amino acid residues.

In one example, the albumin-binding motif comprises the polypeptide sequence of SEQ ID NO.: 9. In one example, the albumin-binding motif comprises a polypeptide sequence having at least 70% sequence identity to the polypeptide sequence of SEQ ID NO.: 9. In one example, the albumin-binding motif comprises a polypeptide sequence having at least 80% sequence identity to the polypeptide sequence of SEQ ID NO.: 9. In one example, the albumin-binding motif comprises a polypeptide sequence having at least 85% sequence identity to the polypeptide sequence of SEQ ID NO.: 9. In one example, the albumin-binding motif comprises a polypeptide sequence having at least 90% sequence identity to the polypeptide sequence of SEQ ID NO.: 9. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 9. In one example, the albumin-binding motif comprises a truncated polypeptide sequence of SEQ ID NO. 9 comprising at least 10, 11, 12, 13, 14, 15, 16, 17, 18, or 19 amino acid residues.

In one example, the albumin-binding motif comprises the polypeptide sequence of SEQ ID NO.: 10. In one example, the albumin-binding motif comprises a polypeptide sequence having at least 70% sequence identity to the polypeptide sequence of SEQ ID NO.: 10. In one example, the albumin-binding motif comprises a polypeptide sequence having at least 80% sequence identity to the polypeptide sequence of SEQ ID NO.: 10. In one example, the albumin-binding motif comprises a polypeptide sequence having at least 85% sequence identity to the polypeptide sequence of SEQ ID NO.: 10. In one example, the albumin-binding motif comprises a polypeptide sequence having at least 90% sequence identity to the polypeptide sequence of SEQ ID NO.: 10. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 10. In one example, the albumin-binding motif comprises a truncated polypeptide sequence of SEQ ID NO. 10 comprising at least 10, 11, 12, 13, 14, 15, 16, 17, or 18 amino acid residues.

In one example, the albumin-binding motif comprises the polypeptide sequence of SEQ ID NO.: 11. In one example, the albumin-binding motif comprises a polypeptide sequence having at least 70% sequence identity to the polypeptide sequence of SEQ ID NO.: 11. In one example, the albumin-binding motif comprises a polypeptide sequence having at least 80% sequence identity to the polypeptide sequence of SEQ ID NO.: 11. In one example, the albumin-binding motif comprises a polypeptide sequence having at least 85% sequence identity to the polypeptide sequence of SEQ ID NO.: 11. In one example, the albumin-binding motif comprises a polypeptide sequence having at least 90% sequence identity to the polypeptide sequence of SEQ ID NO.: 11. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 11. In one example, the albumin-binding motif comprises a truncated polypeptide sequence of SEQ ID NO. 11 comprising at least 10, 11, 12, 13, 14, 15, 16, 17, 18, or 19 amino acid residues.

In one example, the albumin-binding motif comprises the polypeptide sequence of SEQ ID NO.: 12. In one example, the albumin-binding motif comprises a polypeptide sequence having at least 70% sequence identity to the polypeptide sequence of SEQ ID NO.: 12. In one example, the albumin-binding motif comprises a polypeptide sequence having at least 80% sequence identity to the polypeptide sequence of SEQ ID NO.: 12. In one example, the albumin-binding motif comprises a polypeptide sequence having at least 85% sequence identity to the polypeptide sequence of SEQ ID NO.: 12. In one example, the albumin-binding motif comprises a polypeptide sequence having at least 90% sequence identity to the polypeptide sequence of SEQ ID NO.: 12. In one example, the albumin-binding motif comprises a polypeptide sequence having at least 91%, 92%, 93%, 94%, 95%, 96%, 97%, 98%, or 99% sequence identity to the polypeptide sequence of SEQ ID NO.: 12. In one example, the albumin -binding motif comprises a truncated polypeptide sequence of SEQ ID NO. 12 comprising at least 10, 11, 12, 13, 14, 15, 16, 17, 18, or 19 amino acid residues.

In one aspect, an albumin-binding motif having at least 90%, 91%, 92%, 93%, 94%, 95%, 96%, 97%, 98%, 99%, or 100% sequence identity to the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12 contains substitutions (e.g., conservative substitutions), insertions, or deletions relative to the reference sequence. In some embodiments, the albumin-binding motif comprising that sequence retains the ability to bind to albumin. In some embodiments, the albumin-binding motif comprising that sequence retains the ability to bind to albumin when the motif is inserted into a binding agent (e.g., antibody or fusion protein).

The albumin-binding motif(s) may be added to any region of the antibody. In some embodiments, the albumin binding motif is added in the Fc portion of the antibody. In some embodiments, the albumin binding motif is added in the Fab portion of the antibody. In some embodiments, the albumin binding motif is added in one or both heavy chains of the antibody. In some embodiments, the albumin binding motif is added in one or both variable regions of the antibody. In some embodiments, the albumin-binding motif is added in one or both light chains of the antibody. Preferably, the albumin-binding motif is added to a region of the antibody that will not substantially affect the tertiary or quaternary structure of the antibody. More preferably, the albumin-binding motif is added to a region of the antibody that will not substantially affect the antigen binding ability of the antibody. In one embodiment, the albumin-binding motif is added to an amino terminal region of the antibody. In one embodiment, the albumin-binding motif is added to a carboxy terminal region of the antibody.

In one embodiment, multiple albumin-binding motifs are added in the same region (e.g., adjacent to each other or within about 1 to about 30 amino acids of each other) of the binding agent. In one embodiment, multiple albumin-binding motifs are added in different regions of the binding agent (e.g., greater than about 30 amino acids from each other and/or on different polypeptides).

In this invention, the binding agent is an antibody. In some embodiments, the antibody is a non-therapeutic and non-endogenous human antibody. In some embodiments, the antibody is a chimeric antibody, a non-endogenous human antibody, a humanized antibody, or non-human antibody. Antibodies are further defined herein.

In one embodiment, the antibody is a known therapeutic antibody. Antibodies that are contemplated to be modified and used in the present invention include rituximab, trastuzumab, bevacizumab, alemtuzumab, blinatumomab, brentuximab, cetuximab, denosumab, dinutuximab, ibritumomab, ipilimumab, nivolumab, obinutuzumab, ofatumumab, panitumumab, pembrolizumab, pertuzumab, Gemtuzumab, Tositumomab, muromonab, or any biosimilar thereof. For the antibodies that contain an albumin-binding site, one or more additional albumin binding motifs are added to form the modified antibody.

In one aspect, the disclosure relates to an isolated albumin-binding motif. In one example, the isolated albumin-binding motif has at least 80%, 85%, 90%, 91%, 92%, 93%, 94%, 95%, 96%, 97%, 98%, or 99% sequence identity to the polypeptide sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12. In one example, the isolated albumin-binding motif retains the ability to bind albumin. In one example, the isolated albumin-binding motif retains the ability to bind an antibody-binding motif. In one example, the isolated albumin-binding motif retains the ability to bind a peptide having the polypeptide sequence of SEQ ID NO.: 3, SEQ ID NO.: 4, or SEQ ID NO.: 5, or a variant thereof. In one aspect, this disclosure relates to a use of the isolated albumin-binding motif or variant thereof, for example to make a binding agent or other molecule that binds albumin.

Modification and production of peptide sequences, including antibodies, can be performed by any suitable method, including those now known, or developed in the future. This disclosure further relates to polynucleotide sequences that code for the polypeptide sequences of the modified antibodies described herein, as well as cells (e.g., CHO or HEK cells) or non-human animals (e.g., mice) that comprise those polynucleotide sequences.

### Modified Carrier Proteins

In one aspect, this disclosure relates to a modified carrier protein having a modified polypeptide sequence that was modified to comprise at least one antibody-binding motif. In one example, the antibody-binding motif comprises the polypeptide sequence of SEQ ID NO.: 3, SEQ ID NO.:4, or SEQ ID NO.:5. In one embodiment, the modified carrier protein has a higher affinity for an antibody than the carrier protein with an unmodified polypeptide sequence.

In one aspect is disclosed a method for making a modified carrier protein, said method comprising providing a carrier protein having a polypeptide sequence and modifying the polypeptide sequence to comprise an antibody-binding motif. In one example, the modified carrier protein has a higher affinity for binding to an antibody than the carrier protein before modification. In one example, the antibody-binding motif comprises the amino acid sequence of SEQ ID NO.: 3, SEQ ID NO.:4, or SEQ ID NO.:5. In one example, the carrier protein is albumin, ovalbumin, gelatin, elastin, an elastin-derived polypeptide, gliadin, legumin, zein, soy protein, milk protein, or whey protein. In one example, the polypeptide sequence did not comprise the antibody-binding motif prior to modification.

In some embodiments, the carrier protein can be albumin, gelatin, elastin (including topoelastin) or elastin-derived polypeptides (e.g., α-elastin and elastin-like polypeptides (ELPs)), gliadin, legumin, zein, soy protein (e.g., soy protein isolate (SPI)), milk protein (e.g., β-lactoglobulin (BLG) and casein), or whey protein (e.g., whey protein concentrates (WPC) and whey protein isolates (WPI)). In preferred embodiments, the carrier protein is albumin. In preferred embodiments, the albumin is egg white (ovalbumin), bovine serum albumin (BSA), or the like. In even more preferred embodiments, the carrier protein is human serum albumin (HSA). In some embodiments, the carrier protein is a recombinant protein (e.g., recombinant HSA). In some embodiments, the carrier protein is a generally regarded as safe (GRAS) excipient approved by the United States Food and Drug Administration (FDA).

In one example, the antibody-binding motif comprises the polypeptide sequence of SEQ ID NO.: 3. In one example, the antibody-binding motif comprises a polypeptide sequence having at least 70% sequence identity to the polypeptide sequence of SEQ ID NO.: 3. In one example, the antibody-binding motif comprises a polypeptide sequence having at least 80% sequence identity to the polypeptide sequence of SEQ ID NO.: 3. In one example, the antibody-binding motif comprises a polypeptide sequence having at least 85% sequence identity to the polypeptide sequence of SEQ ID NO.: 3. In one example, the antibody-binding motif comprises a polypeptide sequence having at least 90% sequence identity to the polypeptide sequence of SEQ ID NO.: 3. In one example, the antibody-binding motif comprises a polypeptide sequence having at least 91%, 92%, 93%, 94%, 95%, 96%, 97%, 98%, or 99% sequence identity to the polypeptide sequence of SEQ ID NO.: 3. In one example, the antibody-binding motif comprises a truncated polypeptide sequence of SEQ ID NO. 3 comprising at least 5, 6, 7, 8, 9, 10, 11, 12, 13, 14, 15, 16, or 17 amino acid residues.

In one example, the antibody-binding motif comprises the polypeptide sequence of SEQ ID NO.: 4. In one example, the antibody-binding motif comprises a polypeptide sequence having at least 70% sequence identity to the polypeptide sequence of SEQ ID NO.: 4. In one example, the antibody-binding motif comprises a polypeptide sequence having at least 80% sequence identity to the polypeptide sequence of SEQ ID NO.: 4. In one example, the antibody-binding motif comprises a polypeptide sequence having at least 85% sequence identity to the polypeptide sequence of SEQ ID NO.: 4. In one example, the antibody-binding motif comprises a polypeptide sequence having at least 90% sequence identity to the polypeptide sequence of SEQ ID NO.: 4. In one example, the antibody-binding motif comprises a polypeptide sequence having at least 91%, 92%, 93%, 94%, 95%, 96%, 97%, 98%, or 99% sequence identity to the polypeptide sequence of SEQ ID NO.: 4. In one example, the antibody-binding motif comprises a truncated polypeptide sequence of SEQ ID NO. 4 comprising at least 5, 6, 7, 8, 9, 10, 11, 12, 13, 14, 15, 16, or 17 amino acid residues.

In one example, the antibody-binding motif comprises the polypeptide sequence of SEQ ID NO.: 5. In one example, the antibody-binding motif comprises a polypeptide sequence having at least 70% sequence identity to the polypeptide sequence of SEQ ID NO.: 5. In one example, the antibody-binding motif comprises a polypeptide sequence having at least 80% sequence identity to the polypeptide sequence of SEQ ID NO.: 5. In one example, the antibody-binding motif comprises a polypeptide sequence having at least 85% sequence identity to the polypeptide sequence of SEQ ID NO.: 5. In one example, the antibody-binding motif comprises a polypeptide sequence having at least 90% sequence identity to the polypeptide sequence of SEQ ID NO.: 5. In one example, the antibody-binding motif comprises a polypeptide sequence having at least 91%, 92%, 93%, 94%, 95%, 96%, 97%, 98%, or 99% sequence identity to the polypeptide sequence of SEQ ID NO.: 5. In one example, the antibody-binding motif comprises a truncated polypeptide sequence of SEQ ID NO. 5 comprising at least 5, 6, 7, 8, 9, 10, 11, 12, 13, 14, 15, 16, or 17 amino acid residues.

In one aspect, an antibody-binding motif having at least 90%, 91%, 92%, 93%, 94%, 95%, 96%, 97%, 98%, 99%, or 100% sequence identity to the amino acid sequence of SEQ ID NO.: 3, SEQ ID NO.: 4, SEQ ID NO.: 5 contains substitutions (e.g., conservative substitutions), insertions, or deletions relative to the reference sequence. In some embodiments, the antibody-binding motif comprising that sequence retains the ability to bind to an antibody. In some embodiments, the antibody-binding motif comprising that sequence retains the ability to bind to an antibody when the motif is inserted into a carrier protein.

The antibody-binding motif may be added to any region of the carrier protein. Preferably, the antibody-binding motif does not significantly affect the tertiary or quaternary structure of the carrier protein. In one embodiment, the antibody-binding motif is added to an amino terminal region of the carrier protein. In one embodiment, the antibody-binding motif is added to a carboxy terminal region of the carrier protein.

In one embodiment, multiple antibody-binding motifs are added in the same region (e.g., adjacent to each other or within about 1 to about 30 amino acids of each other) of the carrier protein. In one embodiment, multiple antibody-binding motifs are added in different regions of the carrier protein (e.g., greater than about 30 amino acids from each other).

In one example, the present disclosure relates to an isolated antibody-binding motif. In one example, the isolated antibody-binding motif has at least 80%, 85%, 90%, 91%, 92%, 93%, 94%, 95%, 96%, 97%, 98%, or 99% sequence identity to the polypeptide sequence of SEQ ID NO.: 3, SEQ ID NO.: 4, or SEQ ID NO.: 5. In one example, the isolated antibody-binding motif retains the ability to bind antibody. In one example, the isolated antibody-binding motif retains the ability to bind an albumin-binding motif. In one example, the isolated antibody-binding motif retains the ability to bind a peptide having the polypeptide sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12, or a variant thereof. In one aspect, this disclosure relates to a use of the isolated antibody-binding motif or variant thereof, for example to make a carrier protein or other molecule that binds an antibody.

Modification and production of peptide sequences, including carrier proteins, can be performed by any suitable method, including those now known, or those developed in the future. This disclosure further relates to polynucleotide sequences that code for the polypeptide sequences of the modified carrier proteins described herein, as well as cells (e.g., CHO or HEK cells) or non-human animals (e.g., mice) that comprise those polynucleotide sequences.

### Peptide Variants

In certain embodiments, amino acid sequence variants of the antibody-binding motifs and albumin-binding motifs provided herein are contemplated. For example, it may be desirable to improve the binding affinity and/or other biological properties of the motif (or protein containing the motif). Amino acid sequence variants of a motif may be prepared by introducing appropriate modifications into the nucleotide sequence encoding the motif, or by peptide synthesis. Such modifications include, for example, deletions from, and/or insertions into and/or substitutions of residues within the amino acid sequences of the motif. In some embodiments, any combination of deletion, insertion, and substitution can be made to arrive at the final construct, provided that the final construct possesses the desired characteristics, e.g., antibody-binding or albumin-binding.

In certain embodiments, antibody variants having one or more amino acid substitutions are provided. Conservative substitutions are shown in Table 1 under the heading of "preferred substitutions." More substantial changes are provided in Table 1 under the heading of "example substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Example Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gin |
| Asp (D) | Glu; Asn | Glu |
| Cys (C) | Ser; Ala | Ser |
| Gin (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

In certain embodiments, substitutions, insertions, or deletions may occur within the motif so long as such alterations do not substantially reduce the ability of the motif to bind an antibody (for the antibody-binding motif) or albumin (for the albumin-binding motif). For example, conservative alterations (e.g., conservative substitutions as provided herein) that do not substantially reduce binding affinity may be made.

In one embodiment, the variant has between one and five amino acid substitutions at a given position. In one embodiment, the variant has between one and five amino acid deletions at a given position. In one embodiment, the variant has between one and ten amino acid insertions at a given position. In one embodiment, the variant has at least one, for example between one and 200, amino acids added to the carboxy terminal region and/or the amino terminal region.

In one embodiment, an albumin-binding motif variant retains the ability to bind albumin. In one embodiment, the albumin-binding motif variant retains the ability to bind an antibody-binding motif. In one embodiment, the albumin-binding motif variant retains the ability to bind a peptide having the polypeptide sequence of SEQ ID NO.: 3, SEQ ID NO.: 4, or SEQ ID NO.: 5, or a variant thereof.

In one embodiment, an antibody-binding motif variant retains the ability to bind antibody. In one embodiment, the antibody-binding motif variant retains the ability to bind an albumin-binding motif. In one embodiment, the antibody-binding motif variant retains the ability to bind a peptide having the polypeptide sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12, or a variant thereof.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex is used to identify contact points between the antibody and antigen. A crystal structure of an albumin-antibody complex may similarly be used to identify contact points between the antibody and albumin. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues.

### Modified Nanoparticle Complexes

The present disclosure relates to nanoparticle complexes and nanoparticle compositions comprising a carrier protein containing an antibody-binding motif, an antibody or other protein containing an albumin-binding motif, and optionally a therapeutic agent. One or both of the carrier protein and the antibody are modified to contain one or more of the antibody-binding motif and/or albumin-binding motif.

In one example, this disclosure relates to a nanoparticle complex comprising a carrier protein having a modified polypeptide sequence that was modified to comprise at least one antibody-binding motif; antibodies, each antibody having an antigen-binding domain; and optionally a therapeutic agent; wherein the nanoparticle complex has binding specificity for the antigen *in vivo.*

In one example, this disclosure relates to a nanoparticle complex comprising albumin; an antibody or fusion protein having a modified polypeptide sequence that was modified to comprise at least one albumin-binding motif, said antibody or fusion protein having an antigen-binding domain; and optionally a therapeutic agent; wherein the nanoparticle complex has binding specificity for the antigen *in vivo.*

In one example, this disclosure relates to a nanoparticle complex comprising a carrier protein having a modified polypeptide sequence that was modified to comprise at least one antibody-binding motif; an antibody or fusion protein having a modified polypeptide sequence that was modified to comprise at least one albumin-binding motif, said antibody or fusion protein having an antigen-binding domain; and optionally a therapeutic agent; wherein the nanoparticle complex has binding specificity for the antigen *in vivo.*

In one example, this invention relates to a composition comprising nanoparticle complexes as described herein.

In one example, the antibody-binding motif comprises the amino acid sequence of SEQ ID NO.: 3, SEQ ID NO.:4, or SEQ ID NO.:5. In one example, the antibody-binding motif comprises a polypeptide having at least 70%, 80%, 90%, 95%, 96%, 97%, 98%, or 99% sequence identity to the amino acid sequence of SEQ ID NO.: 3, SEQ ID NO.:4, or SEQ ID NO.:5.

In one example, the albumin-binding motif comprises the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12. In one example, the albumin-binding motif comprises a polypeptide having at least 70%, 80%, 90%, 95%, 96%, 97%, 98%, or 99% sequence identity to the amino acid sequence of SEQ ID NO.: 6, SEQ ID NO.: 7, SEQ ID NO.: 8, SEQ ID NO.: 9, SEQ IDNO.: 10, SEQ ID NO.: 11, or SEQ IDNO.: 12.

In one embodiment, the therapeutic agent is paclitaxel.

In one embodiment, the complexes have an average size of less than 1 µm. In one embodiment, the nanoparticle complexes have an average size between about 90 nm and about 800 nm. In one embodiment, the nanoparticle complexes have an average size between about 90 nm and about 400 nm. In one embodiment, the nanoparticle complexes have an average size between about 90 nm and about 200 nm. In one embodiment, the nanoparticle complexes have an average size between about 100 nm and about 800 nm. In one embodiment, the nanoparticle complexes have an average size between about 100 nm and about 400 nm. In one embodiment, the nanoparticle complexes have an average size between about 100 nm and about 200 nm. The size may be any value or subrange within these ranges, including endpoints.

In one embodiment, the ratio of albumin-therapeutic agent to antibody is between 10:1 and 10:30. The ratio may be any value or subrange within this range, including endpoints. In one embodiment, each of the nanoparticle complexes comprises between about 100 and about 1000 antibodies. In one embodiment, each of the nanoparticle complexes comprises between about 100 and about 800 antibodies. The number of antibodies may be any value or subrange within these ranges, including endpoints.

In one embodiment, the albumin is human serum albumin. In one embodiment, the human serum albumin is recombinant human serum albumin.

In one embodiment, the nanoparticle complexes are lyophilized. In one embodiment, the composition comprising nanoparticle complexes is lyophilized. In one embodiment, the lyophilized complex or composition, upon reconstitution in an aqueous solution, comprises nanoparticle complexes that remain capable of binding to (recognize/bind to) the antigen *in vivo.*

In one embodiment, the carrier protein is albumin, ovalbumin, gelatin, elastin, an elastin-derived polypeptide, gliadin, legumin, zein, soy protein, milk protein, or whey protein. In one embodiment, the carrier protein is albumin. In one embodiment, the albumin is modified to contain additional antibody-binding motifs.

In one embodiment, the antibodies or fusion proteins are associated with the carrier protein through non-covalent bonds. In one embodiment, the antibodies or fusion proteins are associated with the carrier protein via the antibody-binding motif and/or the albumin-binding motif. In one embodiment, the paclitaxel is associated with the carrier protein through non-covalent bonds. In one embodiment, wherein the antibodies or fusion proteins are non-covalently associated with the carrier protein via the antibody-binding motif. In one embodiment, the antibodies or fusion proteins are non-covalently associated with the carrier protein via the albumin-binding motif.

In one embodiment, at least a subset of the modified carrier protein comprises more than one antibody-binding motif. In one embodiment, at least a subset of the modified antibodies or fusion proteins comprises more than one albumin-binding motif.

In one embodiment, the composition further comprises a pharmaceutically acceptable excipient.

In some embodiments, the chemotherapeutic agent is associated with a carrier protein. In some embodiments, the complex further comprises a sub-therapeutic amount of paclitaxel.

In some embodiments, the effective amount of the chemotherapeutic agent is selected from an amount consisting of about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², about 150 mg/m², about 155 mg/m², about 160 mg/m², about 165 mg/m², about 170 mg/m², about 175 mg/m², about 180 mg/m², about 185 mg/m², about 190 mg/m², about 195 mg/m², or about 200 mg/m² of the chemotherapeutic drug.

It is to be understood that the therapeutic agent (i.e., chemotherapeutic agent) may be located inside the nanoparticle, on the outside surface of the nanoparticle, or both. The nanoparticle may contain more than one different therapeutic agents, for example, two therapeutic agents, three therapeutic agents, four therapeutic agents, five therapeutic agents, or more. Furthermore, a nanoparticle may contain the same or different therapeutic agents inside and outside the nanoparticle.

In one aspect, the amount of chemotherapeutic agent, e.g. paclitaxel, in the nanoparticle is sufficient to allow formation of the nanoparticle. The use of sub-therapeutic amounts of paclitaxel for formation of antibody-albumin nanoparticle complexes is described, for example, in U.S. Provisional App. No. 62/384,119, filed September 6, 2016.

In one embodiment, the amount of paclitaxel present in the nanoparticle complex is greater than or equal to a minimum amount capable of providing stability to the nanoparticle complex. In one embodiment, the amount of paclitaxel present in the nanoparticle complex is greater than or equal to a minimum amount capable of providing affinity of the at least one therapeutic agent to the protein carrier. In one embodiment, the amount of paclitaxel present in the nanoparticle complex is greater than or equal to a minimum amount capable of facilitating complex-formation of the at least one therapeutic agent and the protein carrier. In one embodiment, the weight ratio of the carrier protein and the paclitaxel of the nanoparticle complex is greater than about 9:1. In one embodiment, the weight ratio is greater than about 10:1, or 11:1, or 12:1, or 13:1, or 14:1, or 15:1, or about 16:1, or about 17:1, or about 18:1, or about 19:1, or about 20:1, or about 21:1, or about 22:1, or about 23:1, or about 24:1, or about 25:1, or about 26:1, or about 27:1, or about 28:1, or about 29:1, or about 30:1. In one embodiment, the amount of paclitaxel is equal to a minimum amount capable of providing stability to the nanoparticle complex. In one embodiment, the amount of paclitaxel is greater than or equal to a minimum amount capable of providing affinity of the at least one therapeutic agent to the protein carrier. In one embodiment, the amount of paclitaxel is greater than or equal to a minimum amount capable of facilitating complex-formation of the at least one therapeutic agent and the protein carrier. In any of these embodiments, the amount of paclitaxel can be less than a therapeutic amount for paclitaxel. In other words, the amount can be less than what is provided or contemplated for providing a therapeutic benefit, such as for example, a chemotherapeutic amount to effectively treat a cancer.

In one embodiment, the amount of paclitaxel present in the nanoparticle composition is less than about 5 mg/mL upon reconstitution with an aqueous solution. In one embodiment, the amount of paclitaxel present in the nanoparticle composition is less than about 4.54 mg/mL, or about 4.16 mg/mL, or about 3.57 mg/mL, or about 3.33 mg/mL, or about 3.12 mg/mL, or about 2.94 mg/mL, or about 2.78 mg/mL, or about 2.63 mg/mL, or about 2.5 mg/mL, or about 2.38 mg/mL, or about 2.27 mg/mL, or about 2.17 mg/mL, or about 2.08 mg/mL, or about 2 mg/mL, or about 1.92 mg/mL, or about 1.85 mg/mL, or about 1.78 mg/mL, or about 1.72 mg/mL, or about 1.67 mg/mL upon reconstitution with an aqueous solution.

In some examples any antibody, aptamer, therapeutic agent, or any combination thereof is expressly excluded.

In some cases, complexes as described herein can be designed to have an average diameter that is less than 1 µm. For example, appropriate concentrations of carrier protein and antibody (or other binding agent) can be used such that complexes having an average diameter that is less than 1 µm are formed. In some cases, the complexes provided herein can have an average diameter that is between 0.1 µm and 1 µm (e.g., between 0.1 µm and 0.95 µm, between 0.1 µm and 0.9 µm, between 0.1 µm and 0.8 µm, between 0.1 µm and 0.7 µm, between 0.1 µm and 0.6 µm, between 0.1 µm and 0.5 µm, between 0.1 µm and 0.4 µm, between 0.1 µm and 0.3 µm, between 0.1 µm and 0.2 µm, between 0.2 µm and 1 µm, between 0.3 µm and 1 µm, between 0.4 µm and 1 µm, between 0.5 µm and 1 µm, between 0.2 µm and 0.6 µm, between 0.3 µm and 0.6 µm, between 0.2 µm and 0.5 µm, or between 0.3 µm and 0.5 µm). Complexes provided herein having an average diameter that is between 0.1 µm and 0.9 µm can be administered systemically (e.g., intravenously) to treat cancer or other disease located within a mammal's body.

In some cases, a complex as provided herein can have greater than 60 percent (e.g., greater than 65, 70, 75, 80, 90, 95, or 99 percent) of the complexes having a diameter that is between 0.1 µm and 0.9 µm (e.g., between 0.1 µm and 0.95 µm, between 0.1 µm and 0.9 µm, between 0.1 µm and 0.8 µm, between 0.1 µm and 0.7 µm, between 0.1 µm and 0.6 µm, between 0.1 µm and 0.5 µm, between 0.1 µm and 0.4 µm, between 0.1 µm and 0.3 µm, between 0.1 µm and 0.2 µm, between 0.2 µm and 1 µm, between 0.3 µm and 1 µm, between 0.4 µm and 1 µm, between 0.5 µm and 1 µm, between 0.2 µm and 0.6 µm, between 0.3 µm and 0.6 µm, between 0.2 µm and 0.5 µm, or between 0.3 µm and 0.5 µm). Complexes provided herein having greater than 60 percent (e.g., greater than 65, 70, 75, 80, 90, 95, or 99 percent) of the complexes with a diameter that is between 0.1 µm and 0.9 µm can be administered systemically (e.g., intravenously) to treat cancer or other disease expressing the relevant antigen located within a mammal's body.

] In one aspect, the average particle size in the nanoparticle composition is less than about 1 µm. In one aspect, the average particle size in the nanoparticle composition is between about 90 nm and about 1 µm, such as between about 90 nm and about 800 nm, between about 90 nm and about 700 nm, between about 90 nm and about 600 nm, between about 90 nm and about 500 nm, between about 90 nm and about 400nm , between about 90 nm and about 300 nm, between about 90 nm and about 200 nm, or between about 90 nm and about 180 nm. Contemplated values include any value, subrange, or range within any of the recited ranges, including endpoints.

In a preferred aspect, the sizes and size ranges recited herein relate to particle sizes of the reconstituted lyophilized nanoparticle composition. That is, after the lyophilized nanoparticles are resuspended in an aqueous solution (e.g., water, PBS, other pharmaceutically acceptable excipient, buffer, etc.), the particle size or average particle size is within the range recited herein.

In one aspect, at least about 50%, 60%, 70%, 80%, 90%, 95%, 96%, 97%, 98%, 99%, 99.5%, or 99.9% of the nanoparticles are present in the reconstituted composition as single nanoparticles. That is, fewer than about 50%, 40%, 30%, etc. of the nanoparticles are dimerized or oligomerized. In some embodiments, the nanoparticles in the composition have less than 20% by number dimerization, less than 10% by number dimerization and preferably less than 5% dimerization.

In some embodiments, the size of the nanoparticle can be controlled by the adjusting the amount (e.g., ratio) of carrier protein to binding agent. The size of the nanoparticles, and the size distribution, is also important. The nanoparticles of the invention may behave differently according to their size. At large sizes, an agglomeration may block blood vessels. Therefore, agglomeration of nanoparticles can affect the performance and safety of the composition. On the other hand, larger particles may be more therapeutic under certain conditions (e.g., when not administered intravenously).

In one aspect, the nanoparticle complex comprises between about 100 and about 1000 binding agents non-covalently bound to the surface of the nanoparticle. In one aspect, the nanoparticle complex comprises between about 200 and about 1000 binding agents non-covalently bound to the surface of the nanoparticle. In one aspect, the nanoparticle complex comprises between about 300 and about 1000 binding agents non-covalently bound to the surface of the nanoparticle. In one aspect, the nanoparticle complex comprises between about 400 and about 1000 binding agents non-covalently bound to the surface of the nanoparticle. In one aspect, the nanoparticle complex comprises between about 500 and about 1000 binding agents non-covalently bound to the surface of the nanoparticle. In one aspect, the nanoparticle complex comprises between about 600 and about 1000 binding agents non-covalently bound to the surface of the nanoparticle. In one aspect, the nanoparticle complex comprises between about 200 and about 800 binding agents non-covalently bound to the surface of the nanoparticle. In one aspect, the nanoparticle complex comprises between about 300 and about 800 binding agents non-covalently bound to the surface of the nanoparticle. In preferred embodiments, the nanoparticle complex comprises between about 400 and about 800 binding agents non-covalently bound to the surface of the nanoparticle. Contemplated values include any value or subrange within any of the recited ranges, including endpoints.

In one embodiment, the binding agents bind to (are specific for) a tumor antigen. In one embodiment, the binding agents bind to Alphafetoprotein (AFP), Carcinoembryonic antigen (CEA), CA-125, MUC-1, Epithelial tumor antigen (ETA), Melanoma-associated antigen (MAGE), Tyrosinase, HER2, HER3, CD3, CD19, CD20, CD33, CD47, CD274, CD279, CD30, CD52, PD-1, PD-L1, CTLA4, GD2, VEGF, BCR-ABL, NY-ESO-1, MAGE-1, MAGE-3, SSX2, Melan-A, EGFR, CD38, or RANK ligand.

### Methods of Making Modified Nanoparticle Complexes

In one aspect, this disclosure relates to a method for making a nanoparticle complex, the method comprising combining albumin with an antibody having a modified polypeptide sequence that was modified to comprise at least one albumin-binding motif under conditions to form the nanoparticle complex. In one example, the carrier protein or the nanoparticle complex is combined with a therapeutic agent. In one example, the therapeutic agent is paclitaxel.

In one aspect is disclosed a method for making a nanoparticle complex, the method comprising incubating a carrier protein having a modified polypeptide sequence that was modified to comprise at least one antibody-binding motif with an antibody. In one example, the carrier protein is albumin. In one example, the carrier protein or the nanoparticle complex is combined with a therapeutic agent. In one example, the therapeutic agent is paclitaxel. In one aspect, the antibody is a modified antibody having a modified polypeptide sequence that was modified to comprise at least one albumin-binding motif.

In one aspect, the nanoparticle complexes are formed by contacting the carrier protein or carrier protein-therapeutic agent particle with the binding agent at a ratio of about 10:1 to about 10:30 carrier protein particle or carrier protein-therapeutic agent particle to binding agent. In one example, the ratio is about 10:2 to about 10:25. In one embodiment, the ratio is about 10:2 to about 1:1. In a preferred example, the ratio is about 10:2 to about 10:6. In an especially preferred example, the ratio is about 10:4. Contemplated ratios include any value, subrange, or range within any of the recited ranges, including endpoints.

In one example, the amount of solution or other liquid medium employed to form the nanoparticles is particularly important. No nanoparticles are formed in an overly dilute solution of the carrier protein (or carrier protein-therapeutic agent) and the antibodies. An overly concentrated solution will result in unstructured aggregates. In some examples, the amount of solution (e.g., sterile water, saline, phosphate buffered saline) employed is between about 0.5 mL of solution to about 20 mL of solution. In some examples, the amount of carrier protein is between about 1 mg/mL and about 100 mg/mL. In some examples, the amount of binding agent is between about 1 mg/mL and about 30 mg/mL. For example, in some examples, the ratio of carrier protein:binding agent:solution is approximately 9 mg of carrier protein (e.g., albumin) to 4 mg of binding agent, e.g., antibody (e.g., BEV) in 1 mL of solution (e.g., saline). An amount of herapeutic agent (e.g., taxol) can also be added to the carrier protein. For example, 1 mg of taxol can be added 9 mg of carrier protein (10 mg carrier protein-therapeutic) and 4 mg of binding agent, e.g., antibody, Fc fusion molecule, or aptamer, in 1 mL of solution. When using a typical i.v. bag, for example, with the solution of approximately 1 liter one would need to use 1000x the amount of carrier protein/carrier protein- therapeutic agent and antibodies compared to that used in 1 mL. Thus, one cannot form the present nanoparticles in a standard i.v. bag. Furthermore, when the components are added to a standard i.v. bag in the therapeutic amounts of the present invention, the components do not self-assemble to form nanoparticles.

In one example, the carrier protein or carrier protein-therapeutic agent particle is contacted with the binding agent in a solution having a pH between about 4 and about 8. In one example, the carrier protein or carrier protein-therapeutic agent particle is contacted with the binding agent in a solution having a pH of about 4. In one embodiment, the carrier protein or carrier protein-therapeutic agent particle is contacted with the binding agent in a solution having a pH of about 5. In one example, the carrier protein or carrier protein-therapeutic agent particle is contacted with the binding agent in a solution having a pH of about 6. In one example, the carrier protein or carrier protein-therapeutic agent particle is contacted with the binding agent in a solution having a pH of about 7. In one example, the carrier protein or carrier protein-therapeutic agent particle is contacted with the binding agent in a solution having a pH of about 8. In a preferred example, the carrier protein or carrier protein-therapeutic agent particle is contacted with the binding agent in a solution having a pH between about 5 and about 7.

In one example, the carrier protein particle or carrier protein-therapeutic agent particle is incubated with the binding agent at a temperature of about 5 °C to about 60 °C, or any range, subrange, or value within that range including endpoints. In a preferred example, the carrier protein particle or carrier protein-therapeutic agent particle is incubated with the binding agent at a temperature of about 23 °C to about 60 °C. In one example, the carrier protein particle or carrier protein-therapeutic agent particle is incubated with the binding agent at room temperature.

Without being bound by theory, it is believed that the stability of the nanoparticle complexes is, at least in part, dependent upon the temperature and/or pH at which the nanoparticle complexes are formed, as well as the concentration of the components *(i.e.,* carrier protein, binding agent, and optionally therapeutic agent) in the solution.

In general, any appropriate combination of carrier protein, chemotherapy agent, and binding agent can be used as described herein. For example, an appropriate amount of carrier protein (e.g., with a chemotherapeutic agent), and an appropriate amount of binding agent can be mixed together in the same container. This mixture can be incubated at an appropriate temperature (e.g., room temperature, between 5 °C and 60 °C, between 23 °C and 60 °C, between 15 °C and 30 °C, between 15 °C and 25 °C, between 20 °C and 30 °C, or between 20 °C and 25 °C) for a period of time (e.g., about 30 minutes, or between about 5 minutes and about 60 minutes, between about 5 minutes and about 45 minutes, between about 15 minutes and about 60 minutes, between about 15 minutes and about 45 minutes, between about 20 minutes and about 400 minutes, or between about 25 minutes and about 35 minutes) before being administered to a patient having a cancer.

In some cases, carrier protein nanoparticles comprising a chemotherapy agent can be contacted with a binding agent to form complexes that are stored prior to being administered to a patient. For example, a composition can be formed as described herein and stored for a period of time (e.g., days or weeks) prior to being administered to a patient.

In some examples, the chemotherapeutic drug is selected from the group consisting of abiraterone, bendamustine, bortezomib, carboplatin, cabazitaxel, cisplatin, chlorambucil, dasatinib, docetaxel, doxorubicin, epirubicin, erlotinib, etoposide, everolimus, gefitinib, idarubicin, imatinib, hydroxyurea, imatinib, lapatinib, leuprorelin, melphalan, methotrexate, mitoxantrone, nedaplatin, nilotinib, oxaliplatin, paclitaxel, pazopanib, pemetrexed, picoplatin, romidepsin, satraplatin, sorafenib, vemurafenib, sunitinib, teniposide, triplatin, vinblastine, vinorelbine, vincristine, and cyclophosphamide.

Both ABRAXANE ^{®} and albumin particles comprising other chemotherapeutic agents are disclosed by U.S. Patent Nos. 7,758,891; 7,820,788; 7,923,536; 8,034,375; 8,138,229; 8,268,348; 8,314,156; 8,853,260; and 9,101,543. In addition, carrier protein, chemotherapeutic drug, antibody conjugates, or combinations thereof are disclosed by PCT/US2015/054295 and U.S. Publication No. 2014/017848.

### Lyophilization

The lyophilized compositions of this invention are prepared by standard lyophilization techniques with or without the presence of stabilizers, buffers, etc. Surprisingly, these conditions do not alter the relatively fragile structure of the nanoparticles. Moreover, at best, these nanoparticles retain their size distribution upon lyophilization and, more importantly, can be reconstituted for *in vivo* administration (e.g., intravenous delivery) in substantially the same form and ratios as if freshly made.

### Formulations

In one aspect, the nanoparticle composition is formulated for systemic delivery, e.g., intravenous administration.

In one aspect, the nanoparticle composition is formulated for direct injection into a tumor. Direct injection includes injection into or proximal to a tumor site, perfusion into a tumor. Because the nanoparticle composition is not administered systemically, a nanoparticle composition is formulated for direct injection into a tumor may comprise any average particle size. Without being bound by theory, it is believed that larger particles (e.g., greater than 500 nm, greater than 1 µm) are more likely to be immobilized within the tumor, thereby providing what is believed to be a better beneficial effect.

In another aspect, provided herein is a composition comprising a compound provided herein, and at least one pharmaceutically acceptable excipient.

In general, the compounds provided herein can be formulated for administration to a patient by any of the accepted modes of administration. Various formulations and drug delivery systems are available in the art. See, *e.g.,* Gennaro, A.R., ed. (1995) Remington's Pharmaceutical Sciences, 18th ed., Mack Publishing Co.

In general, compounds provided herein will be administered as pharmaceutical compositions by any one of the following routes: oral, systemic (e.g., transdermal, intranasal or by suppository), or parenteral (e.g., intramuscular, intravenous or subcutaneous) administration.

The compositions may be comprised of, in general, a compound of the present invention in combination with at least one pharmaceutically acceptable excipient. Acceptable excipients are non-toxic, aid administration, and do not adversely affect the therapeutic benefit of the claimed compounds. Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Preferred liquid carriers, particularly for injectable solutions, include water, saline, aqueous dextrose, and glycols. Other suitable pharmaceutical excipients and their formulations are described in Remington's Pharmaceutical Sciences, edited by E. W. Martin (Mack Publishing Company, 18th ed., 1990).

The present compositions may, if desired, be presented in a pack or dispenser device containing one or more unit dosage forms containing the active ingredient. Such a pack or device may, for example, comprise metal or plastic foil, such as a blister pack, or glass, and rubber stoppers such as in vials. The pack or dispenser device may be accompanied by instructions for administration. Compositions comprising a compound of the invention formulated in a compatible pharmaceutical carrier may also be prepared, placed in an appropriate container, and labeled for treatment of an indicated condition.

### Methods of Using Modified Nanoparticle Complexes

The nanoparticle complexes as described herein are useful in treating cancer cells and/or tumors in a mammal. In a preferred embodiment, the mammal is a human (i.e., a human patient). Preferably, a lyophilized nanoparticle composition is reconstituted (suspended in an aqueous excipient) prior to administration.

In one aspect is provided a method for treating a cancer cell, the method comprising contacting the cell with an effective amount of nanoparticle complexes and an immunotherapy as described herein to treat the cancer cell. Treatment of a cancer cell includes, reduction in proliferation, killing the cell, preventing metastasis of the cell.

Any appropriate method can be used to obtain complexes as described herein. Any appropriate method can be used to administer a complex as provided herein to a mammal. For example, a composition containing carrier protein/binding agent/chemotherapeutic agent complexes can be administered via injection (e.g., subcutaneous injection, intramuscular injection, intravenous injection, or intrathecal injection).

Cancers or tumors that can be treated by the nanoparticle complexes, compositions and methods described herein include, biliary tract cancer; brain cancer, including glioblastomas and medulloblastomas; breast cancer; uterine cancer; tubal cancer; cervical cancer; choriocarcinoma; colon cancer; bladder cancer; endometrial cancer; vaginal cancer; vulvar cancer; esophageal cancer; mouth cancer; gastric cancer; kidney cancer; hematological neoplasms, including acute lymphocytic and myelogenous leukemia; multiple myeloma; AIDS associated leukemias and adult T-cell leukemia lymphoma; intraepithelial neoplasms, including Bowen's disease and Paget's disease; liver cancer (hepatocarcinoma); lung cancer; head or neck cancers or oral cancers (mouth, throat, esophageal, nasopharyngeal, jaw, tonsil, nasal, lip, salivary gland, tongue, etc.); lymphomas, including Hodgkin's disease and lymphocytic lymphomas; neuroblastomas; neuroendocrine tumors; oral cancer, including squamous cell carcinoma; adrenal cancer; anal cancer; angiosarcoma; appendix cancer; bile duct cancer; bone cancer; carcinoid tumors; soft tissue sarcoma; rhabdomyosarcoma; eye cancer; ovarian cancer, including those arising from epithelial cells, stromal cells, germ cells and mesenchymal cells, and fallopian tube cancer; gallbladder cancer; pancreas cancer; prostate cancer; rectal cancer; sarcomas, including leiomyosarcoma, rhabdomyosarcoma, liposarcoma, fibrosarcoma and osteosarcoma; skin cancer, including melanoma, Kaposi's sarcoma, basocellular cancer and squamous cell cancer; testicular cancer, including germinal tumors (seminoma, non-seminoma[teratomas, choriocarcinomas]), stromal tumors and germ cell tumors; penile cancer; hemangioendothelioma; gastrointestinal cancer; ureteral cancer; urethral cancer; spinal cancer; pituitary gland cancer; primary central nervous system (CNS) lymphoma; thyroid cancer, including thyroid adenocarcinoma and medullar carcinoma; and renal cancer including adenocarcinoma and Wilms tumor. In important embodiments, cancers or tumors include breast cancer, prostate cancer, colorectal cancer, lymphoma, multiple myeloma, and melanoma.

Before administering a composition containing a complex as provided herein to a mammal, the mammal can be assessed to determine whether or not the mammal has a cancer or disease expressing the relevant antigen. Any appropriate method can be used to determine whether or not a mammal has a cancer or disease expressing the relevant antigen. For example, a mammal (e.g., human) can be identified using standard diagnostic techniques. In some cases, a tissue biopsy can be collected and analyzed to determine whether or not a mammal has a cancer or disease expressing the antigen.

After identifying a mammal as having the disease or cancer, the mammal can be administered a composition containing a complex as provided herein. For example, a composition containing the complex can be administered prior to or in lieu of surgical resection of a tumor. In some cases, a composition containing a complex as provided herein can be administered following resection of a tumor.

If a particular mammal fails to respond to a particular amount, then the amount can be increased by, for example, two fold. After receiving this higher concentration, the mammal can be monitored for both responsiveness to the treatment and toxicity symptoms, and adjustments made accordingly. The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the cancer or disease may require an increase or decrease in the actual effective amount administered.

A composition containing a complex as provided herein can be administered to a mammal in any appropriate amount, at any appropriate frequency, and for any appropriate duration effective to achieve a desired outcome (e.g., to increase progression-free survival). In some cases, a composition as provided herein can be administered to a mammal having a cancer or disease to reduce the progression rate of the cancer or disease by 5, 10, 25, 50, 75, 100, or more percent. For example, the progression rate can be reduced such that no additional cancer progression is detected.

Any appropriate method can be used to determine whether or not the progression rate of cancer is reduced. For example, the progression rate of a cancer can be assessed by imaging tissue at different time points and determining the amount of cancer cells present. The amounts of cancer cells determined within tissue at different times can be compared to determine the progression rate. After treatment as described herein, the progression rate can be determined again over another time interval. In some cases, the stage of cancer after treatment can be determined and compared to the stage before treatment to determine whether or not the progression rate was reduced.

In some cases, a composition as provided herein can be administered to a mammal having a cancer under conditions where progression-free survival is increased (e.g., by 5, 10, 25, 50, 75, 100, or more percent) as compared to the median progression-free survival of corresponding mammals having untreated cancer or the median progression-free survival of corresponding mammals having cancer treated with the carrier protein, chemotherapy agent, and the binding agent without forming complexes prior to administration. In some cases, a composition as provided herein can be administered to a mammal having a cancer to increase progression-free survival by 5, 10, 25, 50, 75, 100, or more percent as compared to the median progression-free survival of corresponding mammals having a cancer and having received the carrier protein, chemotherapy agent, carrier protein/chemotherapy agent nanoparticle (without a binding agent), or binding agent alone. Progression-free survival can be measured over any length of time (e.g., one month, two months, three months, four months, five months, six months, or longer).

In some cases, a composition containing a complex as provided herein can be administered to a mammal having a under conditions where the 8-week progression-free survival rate for a population of mammals is 65% or greater (e.g., 66%, 67%, 68%, 69%, 70%, 71%, 72%, 73%, 74%, 75%, 76%, 77%, 78%, 79%, 80% or greater) than that observed in a population of comparable mammals not receiving a composition containing complexes as provided herein. In some cases, the composition can be administered to a mammal having a cancer under conditions where the median time to progression for a population of mammals is at least 150 days (e.g., at least 155, 160, 163, 165, or 170 days).

An effective amount of a composition containing complexes as provided herein can be any amount that reduces the progression rate of a cancer or disease expressing the antigen recognized by the binding agent, increases the progression-free survival rate, or increases the median time to progression without producing significant toxicity to the mammal. If a particular mammal fails to respond to a particular amount, then the amount can be increased by, for example, two fold. After receiving this higher concentration, the mammal can be monitored for both responsiveness to the treatment and toxicity symptoms, and adjustments made accordingly. The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the cancer or disease may require an increase or decrease in the actual effective amount administered.

The frequency of administration can be any frequency that reduces the progression rate of a cancer or disease, increases the progression-free survival rate, or increases the median time to progression without producing significant toxicity to the mammal. For example, the frequency of administration can be from about once a month to about three times a month, or from about twice a month to about six times a month, or from about once every two months to about three times every two months. The frequency of administration can remain constant or can be variable during the duration of treatment. A course of treatment with a composition as provided herein can include rest periods. For example, the composition can be administered over a two week period followed by a two week rest period, and such a regimen can be repeated multiple times. As with the effective amount, various factors can influence the actual frequency of administration used for a particular application. For example, the effective amount, duration of treatment, use of multiple treatment agents, route of administration, and severity of the cancer or disease may require an increase or decrease in administration frequency.

An effective duration for administering a composition provided herein can be any duration that reduces the progression rate of a cancer or disease, increases the progression-free survival rate, or increases the median time to progression without producing significant toxicity to the mammal. Thus, the effective duration can vary from several days to several weeks, months, or years. In general, the effective duration for the treatment of a cancer or disease can range in duration from several weeks to several months. In some cases, an effective duration can be for as long as an individual mammal is alive. Multiple factors can influence the actual effective duration used for a particular treatment. For example, an effective duration can vary with the frequency of administration, effective amount, use of multiple treatment agents, route of administration, and severity of the cancer or disease.

A composition containing carrier protein/chemotherapy agent/ binding agent complexes as provided herein can be in any appropriate form. For example, a composition provided herein can be in the form of a solution or powder with or without a diluent to make an injectable suspension. A composition also can contain additional ingredients including pharmaceutically acceptable vehicles. A pharmaceutically acceptable vehicle can be, for example, saline, water, lactic acid, mannitol, or combinations thereof.

After administering a composition provided herein to a mammal, the mammal can be monitored to determine whether or not the cancer or disease was treated. For example, a mammal can be assessed after treatment to determine whether or not the progression rate of the cancer or disease was reduced (e.g., stopped). As described herein, any method can be used to assess progression and survival rates.

### OTHER EMBODIMENTS

It is to be understood that, while the invention has been described in conjunction with the detailed description thereof, the foregoing description is intended to illustrate and not limit the scope of the invention, which is defined by the scope of the appended claims.

### EXAMPLES

One skilled in the art would understand that descriptions of making and using the particles described herein are for the sole purpose of illustration, and that the present disclosure is not limited by this illustration.

Any abbreviation used herein, has normal scientific meaning. All temperatures are °C unless otherwise stated. Herein, the following terms have the following meanings unless otherwise defined:

| | | |
|---|---|---|
| ABX | = | ABRAXANE^{®}/(albumin-bound paclitaxel) |
| ADC | = | antibody dependent chemotherapy |
| BSA | = | bovine serum albumin |
| nM | = | nanomolar |
| nm | = | nanometers |
| EdU | = | 5-ethvnvl-2'-deoxvuridine |
| FITC | = | Fluorescein |
| kD | = | kilo-dalton |
| Kd | = | dissociation constant |
| kg | = | kilogram |
| M | = | molar |
| mg | = | milligram |
| ml or mL | = | milliliter |
| m² | = | square meters |
| mm³ | = | cubic millimeter |
| µg | = | microgram |
| µl or µL | = | microliter |
| µm | = | micrometer/micron |
| PBS | = | Phosphate buffered saline |
| rpm | = | rotations per minute |

### Example 1. Preparation and Characterization of Rituximab-Albumin-Paclitaxel Nanoparticle Complexes

To prepare AR160 nanoparticle complexes, ABRAXANE^{®} (ABX; Celgene, Summit, NJ) and rituximab (Genentech, San Francisco, CA) were mixed at 10 mg/mL and 4 mg/mL, respectively (unless otherwise indicated), and incubated for 30 minutes at room temperature.

Rituximab binds ABRAXANE^{®} (ABX) with a high affinity, with a dissociation constant in the picomolar range. When 4mg/mL of rituximab is mixed with 10mg/mL ABX a 160 nm nanoparticle is formed, AR160. In order to visualize the AR160 nanoparticles, rituximab was labeled with AlexaFluor 488 and incubated with 10 mg/mL ABX. AR160 nanoparticles containing labeled rituximab were visualized using an Amnis ImageStream flow cytometer (FIG. 1A).

To determine rituximab binding to ABX, flow cytometric analysis was performed on AR160 molecules. ABX and rituximab were labeled with Alexa-fluor 488 (Thermo Scientific, Rockford, IL) per manufacturer's protocol. Alexa-fluor 488 was incubated with 1 mg of protein for 60 minutes at room temperature and unbound label was separated from labeled protein by size exclusion column. The AR160 complexes were made as described above, using unlabeled ABX and rituximab, unlabeled ABX and labeled rituximab, or labeled ABX and rituximab. Flow cytometry data are shown in FIG. 1B and show the interaction between ABX and rituximab.

Paclitaxel content of AR160 fractions that included the spun particulate, proteins greater than 100 kD and proteins less than 100 kD was quantitated. AR160 was prepared as described above. The particulate was spun down with two centrifugation steps at 10,000 rpm. About 70% of the paclitaxel remained with the AR160 particulate, and most of the remaining 30% was with proteins greater than 100 kD; a very small percentage (0.3%) of paclitaxel was present in the less than 100 kD protein fraction (FIG. 1C, left panel).

To ascertain the contents of the greater than 100 kD protein fraction, Western blot analysis was performed by staining for paclitaxel, rituximab and human albumin. The protein complexes in the supernatant were denatured then separated by SDS-PAGE gel chromatography. The proteins were then transferred to PVDF membrane and western blotted for albumin with rabbit anti-human albumin at a 1:10,000 dilution (Cell Signaling, Danvers, MA), paclitaxel with rabbit anti-taxol at a 1:10,000 dilution (AbCam, Cambridge, MA), and rituximab with rat anti-rituximab HRP at a 1:500 dilution (Bio-rad, Hercules, CA). Goat anti-rabbit IgG HRP at a dilution of 1:2000 (Cell Signaling, Danvers, MA) was used as a secondary antibody for albumin and taxol. The proteins were visualized by ECL substrate (Thermo Scientific, Rockford, IL).

Albumin, antibody, and paclitaxel co-localize in a band that is approximately 200 kD (FIG. 1C, right panel), which suggests that the 160 nm particle dissociates into functional units that have tumor targeting ability with the antibody and retains the cytotoxic agent within the 200 kD macromolecular species.

### Example 2. Binding of Rituximab-Albumin-Paclitaxel Nanoparticle Complexes to Membrane-Bound CD20

To determine whether AR160 binds to membrane bound CD20, Daudi cells were stained with PE-anti-human CD19 and AR160 in which the ABX was labeled with Alexa fluor 488 and coated with rituximab. Scatterplots show a population of Daudi cells that are 75% positive when stained with PE anti-human CD19, 75% positive when stained with fluorescently tagged AR160, and about 74% double positive when stained with both PE anti-human CD19 and Alexa fluor 488 tagged AR160, suggesting that AR160 binds Daudi cells (FIG. 2A). Stained Daudi cells were also visualized by imaging flow cytometry with the Amnis ImageStream (FIG. 2B).

PE anti-human CD19 and CD20 were purchased from BD Pharmingen (Franklin Lakes, NJ). Daudi cells were incubated for 30 minutes with Alexa-fluor 488 AR160, PE anti-human CD19 and PE anti-human CD20 at 4oC. The cells were washed 2x in FACS buffer (1x PBS and 0.5% BSA with 0.1% NaAzide). The cells were run and data was collected on a Guava flow cytometer (Millipore, Billerica, MA). The flow cytometery data was analyzed using GuavaSoft software (Millipore, Billerica, MA) and percentages of AR160+, CD19+ and CD20+ cells were enumerated. For confocal pictures of Alexa-fluor 488 labeled ABX and PE anti-human CD19/Alexa-fluor 488 AR160 labeled Daudi cells, Amnis ImageStream (Millipore, Billerica, MA) was employed. Inspire software (Millipore, Billerica, MA) was used to analyze data and collect pictures.

### Example 3. Size and Stability of Rituximab-Albumin-Paclitaxel Nanoparticle Complexes

To determine the size of nanoparticle complexes formed using different amounts of rituximab, 10 mg ABX was incubated with 0, 2, 4, 6, 8, or 10 mg rituximab. The Malvern Nanosight (Malvern, Worcestshire, UK) was utilized for size determination. The particles were diluted 1 :200, and a camera level of 9, and capture detection threshold of 16 were used to determine particle size and number. The Nanosight uses light scattering and Brownian motion to obtain particle size and concentration. The resulting sizes are provided in FIG. 2C.

To determine the effect of pH levels during incubation on particle formation, AR160 nanoparticles were formed as indicated in Example 1 at an incubation pH of 3, 7 or 9, and dissociation constants (Kd) determined. The Kd of nanoparticle complexes formed at pH 3 was 4.4 × 10⁻¹⁰; Kd of nanoparticle complexes formed at pH 7 was 3.9 × 10⁻⁹; and Kd of nanoparticle complexes formed at pH 9 was 2.5 × 10⁻⁸. These data indicate that changing the pH of the mixing conditions affects the strength of the ABX/rituximab bond.

The stability of AR160 relative to ABX alone was evaluated using Nanosight technology. ABX and AR160 were made as described and allowed to sit at room temperature in saline for 0 to 6 and 24 hours, and the particle quantity and size were measured at each time point (FIG. 3). The number of particles of ABX alone was 4.23 × 10⁸ compared to AR160, which had a range of 19.1 to 23.5 × 10⁸ particles at 0-24 hours. The mean size of ABX was 90 nm and much smaller than AR160, which had mean sizes of 127-133 nm at 0-24 hours. Additionally, the size of AR160 was stable through 24 hours, as suggested by the number and size of the particles remaining the same throughout the incubation time. ABX became unstable during the incubation time; due to difficulty in measurement of ABX particle size and number, only time 0 is shown (FIG. 3).

To assess the stability of AR160 relative to ABX in serum, the equivalent number (30 × 10⁸) of particles of ABX and AR160 were added to human AB serum. The number of particles remaining at 5, 15, 30 and 60 minutes of incubation at room temperature were quantitated (FIGs. 4A and 4B). At each time point, more AR160 particles were measured (19, 16, 11 and 10× 10⁸) relative to ABX alone (11, 6.6, 4.2, and 5.2 × 10⁸) at 5, 15, 30, and 60 minutes, respectively (FIG. 4C). Furthermore, ABX particle number returned to baseline relative to serum after only 15 minutes of incubation, while AR160 particle numbers remained higher throughout the 60-minute incubation than in serum only (FIG. 4C).

### Example 4. Rituximab-Albumin-Paclitaxel Nanoparticle Complexes Prevent Binding of anti-CD20 antibody to Daudi Cells

Ligand binding capability of the rituximab in the AR160 complex was evaluated. CD20+ Daudi cells were incubated with rituximab (FIG. 5C), ABX (FIG. 5D), AR160 (FIG. 5E), or 24-hour-old AR160 (FIG. 5F). After incubation, the cells were washed and stained with PE-mouse anti-human CD20 and enumerated by flow cytometry. An isotype control (6.2% positive; FIG. 5A) and PE-mouse anti-human CD20 (83.6% positive; FIG. 5B) were used as negative and positive controls, respectively. The results show that rituximab and AR160 prohibit subsequent binding of the anti-human CD20 antibody, suggesting that rituximab alone and in the context of the AR160 complex bind CD20, thereby inhibiting the binding of the anti-CD20 antibody. ABX alone did not inhibit binding of the antibody, indicating that the rituximab in the AR160 particle retains its ligand binding properties in a specific manner.

### Example 5. Toxicity of Rituximab-Albumin-Paclitaxel Nanoparticle Complexes

To ensure that the paclitaxel in AR160 maintained its anti-proliferative capacity, ABX and rituximab alone, AR160, and AR160 that was made 24 hours before testing were tested in an *in vitro* toxicity assay with CD20+ Daudi cells. Cell proliferation was measured with EdU, a thymidine analog, that was detected with a FITC conjugated anti-EdU and enumerated by flow cytometry. The IC50 of all the paclitaxel containing drugs was about 25 µg/mL, while rituximab alone was not toxic (FIG. 6); therefore, paclitaxel toxicity is not compromised in the context of AR160.

The human B-cell lymphoma line, Daudi, (ATCC Manassa, VA) were cultured in RPMI with 1% penicillin, streptomycin and glutamine (PSG) and 10% FBS. Cells were harvested and plated at 0.2 x 106 cells per well in 24 well plates. Cells were exposed to ABX alone or AR160 at paclitaxel concentrations from 0 to 200 µg/mL overnight, or rituximab (0-200 µg/mL) at 37°C and 5% CO2. To measure proliferation, the Click-iT EdU (Molecular Probes, Eugene, OR) kit was utilized. Briefly, 10mM EdU was added to the wells and incubated overnight with the cells and ABX, rituximab or AR160. The cells were permeabilized with 1% saponin and intercalated EdU was labeled with a FITC-conjugated antibody. The proliferation index was determined by dividing the FITC positive cells from each treatment by the maximum proliferation of untreated EdU labeled cells.

### Example 6. In Vivo Testing of Rituximab-Albumin-Paclitaxel Nanoparticle Complexes

To test tumor efficacy, 5 × 10⁶ Daudi human lymphoma cells were implanted into the right flank of athymic nude mice (Harlan Sprague Dawley, Indianapolis, IN). When the tumors had reached a size of about 800 mm³, the mice were randomized and treated with saline, RIT (12 mg/kg; Rit 12), RIT (18 mg/kg; Rit 18) ABX (30 mg/kg; ABX 30), ABX (45 mg/kg; ABX 45), or AR160, which contained 12mg/kg RIT and 30mg/kg ABX (AR160 30) or 18 mg/kg RIT and 45 mg/kg ABX (AR160 45) by intravenous injection of 100 µl in the mouse dorsal tail vein. Tumor size was monitored 2-3 times/week and tumor volume was calculated with the following equation: (length ^{∗} width²)/2. Mice were sacrificed when the tumor size equaled 10% of the mouse body weight, or about 2500 mm³. The day 10 percent change from baseline was calculated as follows: [(tumor size on treatment day-tumor size on_{°} day 10)/tumor size on treatment day]* 100. Kaplan Meier curves were generated and median survival was calculated using GraphPad Prism software (GraphPad Software, Inc, La Jolla, CA).

By day 10, all the mice in the AR160 45 treated group (17/17) had a tumor response while 94.1% (16/17) had complete tumor responses compared to 7/14 (50%), 3/7 (42.8%), 1/4 (25%), 0/7 (0%) and 0/5 (0%) of mice having responses in the AR160 30, ABX 45, ABX 30, RIT 18, RIT 12, and saline groups, respectively (FIGs. 7A-7G). The percentage of mice alive at day 10 in each group were 0%, 12%, 38%, 43%, 71%, 92% and 100% for saline, RIT 12, RIT 18, ABX 30, ABX 45, AR160 30, and AR160 45 groups, respectively (FIG. 7H). The percent change from baseline tumor size in the AR160 45 group compared to all other groups was significant: p<0.0001 for saline and RIT 12, p=0.0003 for RIT 18, p=0.0054 for ABX 30, p=0.0098 for ABX 45 and p=0.003 for AB160 30. The median survival of mice treated with AR160 45 remained undefined at 90 days when all mice were sacrificed compared to 9, 8, 10.5, 12, 16 and 53.5 days for mice treated with saline, RIT 12, RIT 18, ABX 30, ABX 45 and AB160 30, respectively (FIG. 8). The median survival for the AR160 45 group was significantly higher than mice in the saline, RIT 12, RIT 18, ABX 30, ABX 45 (p<0.0001) groups, while the difference between the two AR160 groups was not significant (p=0.0715).

For *in vivo* imaging, Abraxane particles were labeled with AlexaFluor 750 dye following the protocol from the SAIVI Antibody Labeling kit (Thermo Scientific, Rockford, IL). Dye and particle solution was incubated for 60 minutes at room temp and then run through a purification column to remove unbound label. Labeled particles were concentrated using Amicon Ultra centrifugal filters (Millipore, Billerica, MA) to a concentration of 14.54 mg paclitaxel/mL. Abraxane was incubated with IVIG (CSL Berhing, King of Prussia, PA) or rituximab (Genentech, San Francisco, CA) at concentrations of 10 mg/mL and 4 mg/mL, respectively, for 30 minutes. Particle size was checked on Malvern Nanosight (Malvern, Worcestshire, UK) to confirm AR160 formation. Mice were injected with 100 µL of 2 mg/mL of labeled Abraxane, Abraxane incubated with IgG (ABIgG), or AR160. Mice were imaged using a Perkin Elmer IVIS Spectrum (Perkin Elmer, Waltham, MA) at 6, 24, 48, and 72 hours post injection. Fluorescent imagery was done at an excitation/emission spectrum of 710/760 and regions of interest (ROI) applied using living image software (Perkin Elmer, Waltham, MA). Tumor delivery was determined by a measure of average radiant efficiency within the area of the tumor, determined by set ROI's. In comparison of Abraxane, Abx + IgG, and AR160, background ROI's were used to subtract out any effect increased particle stability had on measurements. Background fluorescence was measured using a region of interest (ROI) on the mouse back and subtracted from the fluorescence measurement in the tumor ROI.

After the background was subtracted for each mouse, a 19.1% increase was detected in mice given AR160 relative to ABX alone and ABX bound IgG (FIGs. 9A and 9B), suggesting that addition of the lymphoma-targeting antibody, rituximab, increased deposition of the chemotherapy at the tumor site. Additionally, to show that the increased tumor deposition of ABX in the AR160 treated mice was antibody-ligand mediated, mice were pretreated with 1% (0.12 mg/kg), 10% (1.2 mg/kg) and 100% (12 mg/kg) of the rituximab dosage in AR160 24 hours prior to injecting the fluorescently label AR160. Mice were imaged at 24 hours post AR160 injection (FIG. 9C). The mice injected with AR160 alone had a high level of fluorescently labeled AR160 in the tumor, while the pretreatment with increasing amounts of rituximab showed diminished quantities of labeled AR160 in the tumors (FIG. 9D). Taken together, these data suggest that there are increased levels of labeled AR160 at the tumor site relative to ABX alone, and this increase of drug deposition in the tumor is mediated by the CD20 ligand specificity of the rituximab.

### Example 7. Comparison of Pharmacy-Made Rituximab-Albumin-Paclitaxel Nanoparticle Complexes with Lab-Made Complexes

Three batches of AR160 were prepared by the pharmacy under the conditions determined in the lab (AR160 p1, p2 and p3). These were compared to ABX alone and lab-prepared AR160 (AR160) and analyzed for size distribution by NanoSight, as well as for particle number in each preparation (FIG. 10). AR160 sizes and particle numbers were similar, regardless of preparation.

Ligand binding capability of the rituximab in the AR160 complexes was evaluated as described in Example 4. Each pharmacy-made batch prevented anti-CD20 antibody binding to Daudi cells in a manner similar to lab-made AR160 (FIGs. 11A- I).

### Example 8. Determination of Antibody-Binding Motif of Human Serum Albumin

Biacore surface plasmon resonance technology was utilized to determine where the binding sites between various antibodies (rituximab, trastuzumab, bevacizumab, and muromonab) and albumin were located. An albumin peptide library was constructed with 18 amino acid peptides, each containing a 6 amino acid overlap, and each albumin peptide was run against rituximab fixed on a CM5 chip. Peptides were suspended to 5-10 mg/mL in HBS-EP plus running buffer. Water insoluble peptides were dissolved in 10% DMSO (Sigma-Aldrich, St. Louis, MO). Rituximab was immobilized onto Biacore CM5 (GE Healthcare, Chicago, IL) chips via amine coupling. Biacore X-100 (GE Healthcare, Chicago, IL) was used to screen the albumin peptide libraries over immobilized rituximab. Peptides were screened from 1-50 µg/mL with an exposure time of 120 seconds. Biacore X100 software was used to determine binding kinetics.

Peptides were run at 50, 25, 10, 5, 2.5, 1.25 µg/mL in HBS running buffer. Dissociation constants were determined by Biacore Evaluation Software. Three albumin peptides were found to bind the antibodies: HSA peptide 4 (SEQ ID NO.: 3), HSA peptide 13 (SEQ ID NO.: 4), and HSA peptide 40 (SEQ ID NO.: 5) (FIGs. 12A-12J). Dissociation constants (Kd) are provided in FIG. 12K. Interestingly, peptide 40 maps to the well-characterized Sudlow II site, a known hydrophobic binding site that binds many drugs. Diana, F.J., Veronich, K. & Kapoor, A.L. Binding of nonsteroidal anti-inflammatory agents and their effect on binding of racemic warfarin and its enantiomers to human serum albumin. J Pharm Sci 78, 195-199 (1989); Sudlow, G., Birkett, D.J. & Wade, D.N. The characterization of two specific drug binding sites on human serum albumin. Mol Pharmacol 11, 824-832 (1975).

### Example 9. Determination of Albumin-Binding Motif of Multiple Antibodies

Biacore surface plasmon resonance technology was also utilized to determine the site on rituximab, trastuzumab, bevacizumab or muromonab that binds to HSA peptide 4, 13, or 40 (FIGs. 13A-13E). The sequence of the variable region of the heavy chain where the binding site is located is provided in FIG. 13F for both bevacizumab (SEQ ID NO.: 1) and rituximab (SEQ ID NO.: 2). The 19-AA region of interest is underlined for bevacizumab (SEQ ID NO.: 8 - WYFDVWGQGTLVTVSSAST) and rituximab (SEQ ID NO.: 10 - WYFNVWGAGTTVTVSAAST), and share about 80% identity. FIG. 13G provides the sequences from each antibody that bind HSA. Interestingly, while muromonab bound to HSA peptides 4 and 40, the muromonab peptide did not bind to the full HSA protein.

As discussed above, co-incubation of 4 mg/mL rituximab with 10 mg/mL of ABX results in a shift of size of the 80 nm ABX alone to approximately 110 nM when rituximab is bound to ABX, as determined by NanoSight. Albumin binding peptides were used in a competition assay to see if they would interfere with the formation of AR160. Briefly, 10 mg/ml of ABX was incubated for 30 minutes with 4 mg/mL of rituximab and a 10 molar excess of either a control peptide (HSA 10), HSA Peptide 40, or no peptide (AR160 Control). After incubation for all particle sizing and enumeration, the Malvern Nanosight (Malvern, Worcestshire, UK) was utilized. The particles were diluted 1:200, and a camera level of 9, and capture detection threshold of 16 were used to determine particle size and number.

ABX alone had a size of 77 nm, while rituximab binding to ABX yielded 100 nm particles. When the albumin binding peptide (peptide 40) was added to an incubation of ABX and with rituximab, the resultant nanoparticle was 70 nm, the size of ABX alone, while a non-binding control peptide resulted in 100 nm particles, the size of AR160 (109 nm) in this experiment (FIG. 14A). These data suggest that HSA peptide 40 effectively inhibited the formation of AR160. The results with the binding peptides 4 and 13 demonstrated a more heterogenous nanoparticle population, suggesting that these 2 albumin peptides, which have less affinity for rituximab, incompletely blocked the formation of AR160 (FIGs. 14C and 14D).

A similar experiment was performed using bevacizumab (FIG. 14B). Results show that addition of HSA Peptide 40 or Bev Peptide 1 (SEQ ID NO.: 7) prevent the 30 nm increase in particle size indicative of antibody complexation with ABX, indicating that both peptides interfere with antibody-albumin binding.

## Claims

1. A composition comprising nanoparticle complexes, wherein each of the nanoparticle complexes comprises:
a carrier protein having at least one antibody-binding motif inserted, wherein the antibody-binding motif consists of an amino acid sequence having at least about 80% sequence identity to the amino acid sequence of SEQ ID NO.: 3, SEQ ID NO.:4, or SEQ ID NO.:5, optionally wherein at least a subset of the carrier protein comprises more than one antibody-binding motif;
antibodies, each antibody having an antigen-binding domain; and
optionally a therapeutic agent;
wherein the nanoparticle complexes have binding specificity for the antigen *in vivo,* optionally wherein each of the nanoparticle complexes comprises between about 100 and about 1000 antibodies.

2. A composition comprising nanoparticle complexes, wherein each of the nanoparticle complexes comprises:
albumin, optionally human serum albumin;
an antibody or fusion protein having at least one albumin-binding motif inserted, wherein the albumin-binding motif consists of an amino acid sequence having at least about 80% sequence identity to the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12, said antibody or fusion protein having an antigen-binding domain, optionally wherein at least a subset of the antibody or fusion protein comprises more than one albumin-binding motif; and
optionally a therapeutic agent;
wherein the nanoparticle complexes have binding specificity for the antigen *in vivo.*

3. A composition comprising nanoparticle complexes, wherein each of the nanoparticle complexes comprises:
a carrier protein having at least one antibody-binding motif inserted, wherein the antibody-binding motif consists of an amino acid sequence having at least about 80% sequence identity to the amino acid sequence of SEQ ID NO.: 3, SEQ ID NO.:4, or SEQ ID NO.:5, optionally wherein at least a subset of the carrier protein comprises more than one antibody-binding motif;
an antibody or fusion protein having at least one albumin-binding motif inserted, wherein the albumin-binding motif consists of an amino acid sequence having at least about 80% sequence identity to the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12, optionally wherein at least a subset of the antibody or fusion protein comprises more than one albumin-binding motif, said antibody or fusion protein having an antigen-binding domain; and
optionally a therapeutic agent;
wherein the nanoparticle complexes have binding specificity for the antigen *in vivo.*

4. The composition of claim 1 or 3, wherein the antibody-binding motif consists of an amino acid sequence having at least about 95% sequence identity to the amino acid sequence of SEQ ID NO.: 3, SEQ ID NO.:4, or SEQ ID NO: 5.

5. The composition of claim 2 or 3, wherein the albumin-binding motif consists of an amino acid sequence having at least about 95% sequence identity to the amino acid sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12.

6. The composition of claim 1 or 3, wherein the carrier protein is albumin, ovalbumin, gelatin, elastin, alpha-elastin, gliadin, legumin, zein, beta-lactoglobulin, or casein, optionally wherein the ratio of carrier protein-therapeutic agent to antibody or fusion protein is between 10:1 and 10:30.

7. The composition of claim 2, wherein the ratio of albumin-therapeutic agent to antibody or fusion protein is between 10:1 and 10:30.

8. The composition of any one of claims 1-7, wherein the therapeutic agent is paclitaxel.

9. The composition of any one of claims 1-8, wherein said complexes have an average size of less than 1 µm, optionally between about 100 nm and about 800 nm.

10. The composition of any one of claims 1-9, which is lyophilized.

11. The composition of claim 1 or 3, wherein the antibody or fusion protein is associated with the carrier protein through non-covalent bonds, optionally via the antibody-binding motif and/or the albumin-binding motif, and/or wherein the paclitaxel is associated with the carrier protein through non-covalent bonds.

12. The composition of any one of claims 1-11, further comprising a pharmaceutically acceptable excipient.

13. The nanoparticle composition of any one of claims 1-12 for use in treating a cancer in a patient, wherein the cancer expresses the antigen.

14. An antibody having at least one albumin-binding motif inserted, wherein the albumin-binding motif consists of the polypeptide sequence of SEQ ID NO.: 6, SEQ ID NO.: 7, SEQ ID NO.: 8, SEQ ID NO.: 9, SEQ ID NO.: 10, SEQ ID NO.: 11, or SEQ ID NO.: 12, optionally wherein the antibody has a higher affinity for albumin than the antibody without an inserted albumin-binding motif.

15. A carrier protein having at least one antibody-binding motif inserted, wherein the antibody-binding motif consists of the polypeptide sequence of SEQ ID NO.: 3, SEQ ID NO.:4, or SEQ ID NO.:5, optionally wherein the carrier protein has a higher affinity for an antibody than the carrier protein without an inserted antibody-binding motif.

## Patentansprüche

1. Zusammensetzung, umfassend Nanopartikelkomplexe, wobei jeder der Nanopartikelkomplexe umfasst:
ein Trägerprotein, das mindestens ein Antikörper-bindendes Motiv eingefügt hat, wobei das Antikörper-bindende Motiv aus einer Aminosäuresequenz besteht, die mindestens etwa 80 % Sequenzidentität mit der Aminosäuresequenz von SEQ ID NO.:3, SEQ ID NO.:4 oder SEQ ID NO.:5 aufweist, wobei gegebenenfalls mindestens eine Teilmenge des Trägerproteins mehr als ein Antikörper-bindendes Motiv umfasst;
Antikörper, wobei jeder Antikörper eine Antigen-bindende Domäne aufweist; und
gegebenenfalls ein Therapeutikum;
wobei die Nanopartikelkomplexe eine Bindungsspezifität für das Antigen *in vivo* aufweisen, gegebenenfalls wobei jeder der Nanopartikelkomplexe zwischen etwa 100 und etwa 1000 Antikörper umfasst.

2. Zusammensetzung, umfassend Nanopartikelkomplexe, wobei jeder der Nanopartikelkomplexe umfasst:
Albumin, gegebenenfalls humanes Serumalbumin;
einen Antikörper oder ein Fusionsprotein, der/das mindestens ein Albumin-bindendes Motiv eingefügt hat, wobei das Albumin-bindende Motiv aus einer Aminosäuresequenz besteht, die mindestens etwa 80 % Sequenzidentität mit der Aminosäuresequenz von SEQ ID NO.:6, SEQ ID NO.:7, SEQ ID NO.:8, SEQ ID NO.:9, SEQ ID NO.:10, SEQ ID NO.:11 oder SEQ ID NO.:12 aufweist, wobei der Antikörper oder das Fusionsprotein eine Antigen-bindende Domäne aufweist, wobei gegebenenfalls mindestens eine Teilmenge des Antikörpers oder Fusionsproteins mehr als ein Albumin-bindendes Motiv umfasst; und
gegebenenfalls ein Therapeutikum;
wobei die Nanopartikelkomplexe eine Bindungsspezifität für das Antigen *in vivo* aufweisen.

3. Zusammensetzung, umfassend Nanopartikelkomplexe, wobei jeder der Nanopartikelkomplexe umfasst:
ein Trägerprotein, das mindestens ein Antikörper-bindendes Motiv eingefügt hat, wobei das Antikörper-bindende Motiv aus einer Aminosäuresequenz besteht, die mindestens etwa 80 % Sequenzidentität mit der Aminosäuresequenz von SEQ ID NO.:3, SEQ ID NO.:4 oder SEQ ID NO.:5 aufweist, wobei gegebenenfalls mindestens eine Teilmenge des Trägerproteins mehr als ein Antikörper-bindendes Motiv umfasst;
einen Antikörper oder ein Fusionsprotein, der/das mindestens ein Albumin-bindendes Motiv eingefügt hat, wobei das Albumin-bindende Motiv aus einer Aminosäuresequenz besteht, die mindestens etwa 80 % Sequenzidentität mit der Aminosäuresequenz von SEQ ID NO.:6, SEQ ID NO.:7, SEQ ID NO.:8, SEQ ID NO.:9, SEQ ID NO.:10, SEQ ID NO.:11 oder SEQ ID NO.:12 aufweist, gegebenenfalls wobei mindestens eine Teilmenge des Antikörpers oder Fusionsproteins mehr als ein Albumin-bindendes Motiv umfasst, wobei der Antikörper oder das Fusionsprotein eine Antigen-bindende Domäne aufweist; und
gegebenenfalls ein Therapeutikum;
wobei die Nanopartikelkomplexe eine Bindungsspezifität für das Antigen *in vivo* aufweisen.

4. Zusammensetzung nach Anspruch 1 oder 3, wobei das Antikörper-bindende Motiv aus einer Aminosäuresequenz besteht, die mindestens etwa 95 % Sequenzidentität mit der Aminosäuresequenz von SEQ ID NO.:3, SEQ ID NO.:4 oder SEQ ID NO.:5 aufweist.

5. Zusammensetzung nach Anspruch 2 oder 3, wobei das Albumin-bindende Motiv aus einer Aminosäuresequenz besteht, die mindestens etwa 95 % Sequenzidentität mit der Aminosäuresequenz von SEQ ID NO.:6, SEQ ID NO.:7, SEQ ID NO.:8, SEQ ID NO.:9, SEQ ID NO.:10, SEQ ID NO.:11 oder SEQ ID NO.:12 aufweist.

6. Zusammensetzung nach Anspruch 1 oder 3, wobei das Trägerprotein Albumin, Ovalbumin, Gelatine, Elastin, Alpha-Elastin, Gliadin, Legumin, Zein, Beta-Lactoglobulin oder Casein ist, wobei gegebenenfalls das Verhältnis von Trägerprotein-Therapeutikum zu Antikörper oder Fusionsprotein zwischen 10:1 und 10:30 liegt.

7. Zusammensetzung nach Anspruch 2, wobei das Verhältnis von Albumin-Therapeutikum zu Antikörper oder Fusionsprotein zwischen 10:1 und 10:30 liegt.

8. Zusammensetzung nach einem der Ansprüche 1-7, wobei das Therapeutikum Paclitaxel ist.

9. Zusammensetzung nach einem der Ansprüche 1-8, wobei die Komplexe eine durchschnittliche Größe von weniger als 1 µm, gegebenenfalls zwischen etwa 100 nm und etwa 800 nm, aufweisen.

10. Zusammensetzung nach einem der Ansprüche 1-9, die lyophilisiert ist.

11. Zusammensetzung nach Anspruch 1 oder 3, wobei der Antikörper oder das Fusionsprotein mit dem Trägerprotein über nicht-kovalente Bindungen, gegebenenfalls über das Antikörper-bindende Motiv und/oder das Albumin-bindende Motiv, verbunden ist und/oder wobei das Paclitaxel mit dem Trägerprotein über nicht-kovalente Bindungen verbunden ist.

12. Zusammensetzung nach einem der Ansprüche 1-11, ferner umfassend einen pharmazeutisch akzeptablen Hilfsstoff.

13. Nanopartikelzusammensetzung nach einem der Ansprüche 1-12 zur Verwendung bei der Behandlung eines Krebses bei einem Patienten, wobei der Krebs das Antigen exprimiert.

14. Antikörper, der mindestens ein Albumin-bindendes Motiv eingefügt hat, wobei das Albumin-bindende Motiv aus der Polypeptidsequenz von SEQ ID NO.:6, SEQ ID NO.:7, SEQ ID NO.:8, SEQ ID NO.:9, SEQ ID NO.:10, SEQ ID NO.:11 oder SEQ ID NO.:12 besteht, wobei der Antikörper gegebenenfalls eine höhere Affinität für Albumin aufweist als der Antikörper ohne ein eingefügtes Albumin-bindendes Motiv.

15. Trägerprotein, das mindestens ein Antikörper-bindendes Motiv eingefügt hat, wobei das Antikörper-bindende Motiv aus der Polypeptidsequenz von SEQ ID NO.:3, SEQ ID NO.:4 oder SEQ ID NO.:5 besteht, wobei das Trägerprotein gegebenenfalls eine höhere Affinität für einen Antikörper aufweist als das Trägerprotein ohne eingefügtes Antikörper-bindendes Motiv.

## Revendications

1. Composition comprenant des complexes de nanoparticules, dans laquelle chacun des complexes de nanoparticules comprend :
une protéine porteuse ayant au moins un motif de liaison à l'anticorps inséré, le motif de liaison à l'anticorps consistant en une séquence d'acides aminés ayant au moins environ 80 % d'identité de séquence avec la séquence d'acides aminés de SEQ ID NO.:3, SEQ ID NO.:4, ou SEQ ID NO.:5, éventuellement au moins un sous-ensemble de la protéine porteuse comprenant plus d'un motif de liaison à l'anticorps ;
des anticorps, chaque anticorps ayant un domaine de liaison à l'antigène ; et
éventuellement un agent thérapeutique ;
dans laquelle les complexes de nanoparticules ont une spécificité de liaison pour l'antigène *in vivo,* éventuellement chacun des complexes de nanoparticules comprend entre environ 100 et environ 1 000 anticorps.

2. Composition comprenant des complexes de nanoparticules, dans laquelle chacun des complexes de nanoparticules comprend :
l'albumine, éventuellement la sérumalbumine humaine ;
un anticorps ou une protéine de fusion ayant au moins un motif de liaison à l'albumine inséré, le motif de liaison à l'albumine consistant en une séquence d'acides aminés ayant au moins environ 80 % d'identité de séquence avec la séquence d'acides aminés de SEQ ID NO.:6, SEQ ID NO.:7, SEQ ID NO.:8, SEQ ID NO.:9, SEQ ID NO.:10, SEQ ID NO.:11, ou SEQ ID NO.:12, ledit anticorps ou ladite protéine de fusion ayant un domaine de liaison à l'antigène, éventuellement au moins un sous-ensemble de l'anticorps ou de la protéine de fusion comprenant plus d'un motif de liaison à l'albumine ; et
éventuellement un agent thérapeutique ;
dans laquelle les complexes de nanoparticules ont une spécificité de liaison pour l'antigène *in vivo.*

3. Composition comprenant des complexes de nanoparticules, dans laquelle chacun des complexes de nanoparticules comprend :
une protéine porteuse ayant au moins un motif de liaison à l'anticorps inséré, le motif de liaison à l'anticorps consistant en une séquence d'acides aminés ayant au moins environ 80 % d'identité de séquence avec la séquence d'acides aminés de SEQ ID NO.:3, SEQ ID NO.:4, ou SEQ ID NO.:5, éventuellement au moins un sous-ensemble de la protéine porteuse comprenant plus d'un motif de liaison à l'anticorps ;
un anticorps ou une protéine de fusion ayant au moins un motif de liaison à l'albumine inséré, le motif de liaison à l'albumine consistant en une séquence d'acides aminés ayant au moins environ 80 % d'identité de séquence avec la séquence d'acides aminés de SEQ ID NO.:6, SEQ ID NO.:7, SEQ ID NO.:8, SEQ ID NO.:9, SEQ ID NO.:10, SEQ ID NO.:11, ou SEQ ID NO.:12, éventuellement au moins un sous-ensemble de l'anticorps ou la protéine de fusion comprenant plus d'un motif de liaison à l'albumine, ledit anticorps ou ladite protéine de fusion ayant un domaine de liaison à l'antigène ; et
éventuellement un agent thérapeutique ;
dans laquelle les complexes de nanoparticules ont une spécificité de liaison pour l'antigène *in vivo.*

4. Composition selon la revendication 1 ou 3, dans laquelle le motif de liaison à l'anticorps consiste en une séquence d'acides aminés ayant au moins environ 95 % d'identité de séquence avec la séquence d'acides aminés de SEQ ID NO.:3, SEQ ID NO.:4, ou SEQ ID NO.:5.

5. Composition selon la revendication 2 ou 3, dans laquelle le motif de liaison à l'albumine consiste en une séquence d'acides aminés ayant au moins environ 95 % d'identité de séquence avec la séquence d'acides aminés de SEQ ID NO.:6, SEQ ID NO.:7, SEQ ID NO.:8, SEQ ID NO.:9, SEQ ID NO.:10, SEQ ID NO.:11 ou SEQ ID NO.:12.

6. Composition selon la revendication 1 ou 3, dans laquelle la protéine porteuse est l'albumine, l'ovalbumine, la gélatine, l'élastine, l'alpha-élastine, la gliadine, la légumine, la zéine, la bêta-lactoglobuline ou la caséine, éventuellement dans laquelle le rapport protéine porteuse-agent thérapeutique à l'anticorps ou à la protéine de fusion étant compris entre 10:1 et 10:30.

7. Composition selon la revendication 2, dans laquelle le rapport de l'albumine-agent thérapeutique à l'anticorps ou à la protéine de fusion est compris entre 10:1 et 10:30.

8. Composition selon l'une quelconque des revendications 1 à 7, dans laquelle l'agent thérapeutique est le paclitaxel.

9. Composition selon l'une quelconque des revendications 1 à 8, dans laquelle lesdits complexes ont une taille moyenne inférieure à 1 µm, éventuellement entre environ 100 nm et environ 800 nm.

10. Composition selon l'une quelconque des revendications 1 à 9, qui est lyophilisée.

11. Composition selon la revendication 1 ou 3, dans laquelle l'anticorps ou la protéine de fusion est associé(e) à la protéine porteuse par des liaisons non covalentes, éventuellement via le motif de liaison à l'anticorps et/ou le motif de liaison à l'albumine, et/ou dans laquelle le paclitaxel est associé à la protéine porteuse par des liaisons non covalentes.

12. Composition selon l'une quelconque des revendications 1 à 11, comprenant en outre un excipient pharmaceutiquement acceptable.

13. Composition de nanoparticules selon l'une quelconque des revendications 1 à 12 destinée à être utilisée dans le traitement d'un cancer chez un patient, le cancer exprimant l'antigène.

14. Anticorps ayant au moins un motif de liaison à l'albumine inséré, le motif de liaison à l'albumine consistant en la séquence polypeptidique de SEQ ID NO.:6, SEQ ID NO.:7, SEQ ID NO.:8, SEQ ID NO.:9, SEQ ID NO.:10, SEQ ID NO.:11, ou SEQ ID NO.:12, éventuellement l'anticorps ayant une plus grande affinité pour l'albumine que l'anticorps sans motif de liaison à l'albumine inséré.

15. Protéine porteuse ayant au moins un motif de liaison à l'anticorps inséré, dans laquelle le motif de liaison à l'anticorps consiste en la séquence polypeptidique de SEQ ID NO.:3, SEQ ID NO.:4 ou SEQ ID NO.:5, éventuellement la protéine porteuse ayant une plus grande affinité pour un anticorps que la protéine porteuse sans motif de liaison à l'anticorps inséré.
